# EUROPÄISCHE PATENTSCHRIFT

(11) **EP 4 753 780 B1**
(45) Veröffentlichungstag und Bekanntmachung des Hinweises auf die Patenterteilung: **04.11.2026**
(21) Anmeldenummer: 24768514.2
(22) Anmeldetag: 06.09.2024
(51) Int. Cl.: A61M 16/04

(54) **TRACHEALKANÜLE**
TRACHEAL CANNULA
CANULE TRACHÉALE

(30) Priorität: 07.09.2023 DE 102023124087
(43) Veröffentlichungstag der Anmeldung: 10.06.2026
(73) Patentinhaber: Andreas Fahl Medizintechnik-Vertrieb GmbH, 51149 Köln (DE)
(72) Erfinder: FAHL, Andreas, 51149 Köln (DE); BENDER, Thomas, 53804 Much (DE)
(74) Vertreter: Geskes, Christoph
(86) Internationale Anmeldenummer: PCT/EP2024/074908
(87) Internationale Veröffentlichungsnummer: WO 2025/051904

(56) Entgegenhaltungen:
- DE-A1- 2 155 088
- US-B2- 10 864 339

## Beschreibung

Die Erfindung betrifft eine Trachealkanüle.

Trachealkanülen sind aus dem Stand der Technik allgemein bekannt. So offenbart DE 20 2017 002 009 U1 ein System umfassend eine Trachealkanüle und eine Einführhilfe, wobei Einführhilfe und Trachealkanüle formschlüssig koppelbar sind.

US 10,864,339 B2 offenbart einen Beatmungsschlauch, dessen Konstruktion einen gleichbleibenden Innendurchmesser vorsieht. DE 2 155 088 A1 offenbart eine Ersatzluftröhre zum Einsetzen in die natürliche Luftröhre zur Unterstützung der Atmung mit einer Kanüle. Nachteilig an dem Stand der Technik ist, dass die Einführung der Trachealkanüle meist traumatisch erfolgt. So weisen bekannte Trachealkanülen einen Absatz zwischen einer Spitze eines Obturators und einem Endabschnitt der Trachealkanüle auf. Weiterhin nachteilig ist die Wandung und/oder an die Wandung angebrachte Schläuche bei der Einführung der Trachealkanüle hinderlich. Weiterhin nachteilhaft sind Kanülenschilde, die nicht ausreichend an die Anatomie des Benutzers anpassbar sind.

Aufgabe der vorliegenden Erfindung ist es, eine verbesserte Trachealkanüle zur Verfügung zu stellen.

Die Aufgabe wird erfindungsgemäß gelöst mittels einer Trachealkanüle zumindest umfassend ein Kanülenrohr und ein Kanülenschild, wobei das Kanülenrohr einen distalen Endabschnitt, dem das Kanülenschild zugeordnet ist, und einen trachealen Endabschnitt umfasst, der in einer Trachea anordenbar ist, wobei dem Kanülenrohr ein erster Außendurchmesser am Kanülenschild zugeordnet ist, wobei dem Kanülenrohr ein zweiter Außendurchmesser an einem trachealen Ende zugeordnet ist, wobei das Kanülenrohr eine von einem distalen Ende zu einem trachealen Ende durchgehende Kanülenausnehmung mit einem gleichbleibenden Innendurchmesser umfasst, wobei eine Wandung zumindest des tracheale Endabschnitts eine erste Wandstärke angrenzend an den distalen Endabschnitt und eine zweite Wandstärke angrenzend am trachealen Ende umfasst, wobei die erste Wandstärke der Wandung an der Stelle des ersten Außendurchmessers größer als eine zweite Wandstärke der Wandung an der Stelle des zweiten Außendurchmessers ist, wobei die erste Wandstärke größer ist als die zweite Wandstärke ist und die Wandstärke des trachealen Endabschnitts zum trachealen Ende hin kontinuierlich abnimmt.

Es wird eine Trachealkanüle zumindest umfassend ein Kanülenrohr und ein Kanülenschild vorgeschlagen. Das Kanülenrohr umfasst einen distalen Endabschnitt, dem das Kanülenschild zugeordnet ist, und einen trachealen Endabschnitt, der in einer Trachea anordenbar ist. Das Kanülenrohr umfasst eine von einem distalen Ende zu einem trachealen Ende durchgehende Kanülenausnehmung mit einem gleichbleibenden Innendurchmesser. Eine Wandung zumindest des tracheale Endabschnitts umfasst eine erste Wandstärke, die an den distalen Endabschnitt angrenzt, und eine zweite Wandstärke am trachealen Ende, wobei die erste Wandstärke größer ist als die zweite Wandstärke ist. Die Wandstärke des trachealen Endabschnitts nimmt zum trachealen Ende hin bevorzugt über die gesamte Länge des trachealen Endabschnitts ab. Die Wandstärke des trachealen Endabschnitts nimmt zum trachealen Ende hin kontinuierlich ab.

In einer Ausgestaltung ist vorgesehen, dass das Kanülenrohr eine Wandung umfasst, die zumindest eine erste Schlauchführung umfasst, in der zumindest ein erster Schlauch angeordnet ist, wobei zumindest die erste Schlauchführung zumindest abschnittsweise als Nut in der Wandung ausgebildet ist, die zumindest abschnittsweise eine Tiefe umfasst, die größer als oder in etwa gleich einem Außendurchmesser des ersten Schlauches ist.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 % vorgesehen.

Soweit verschiedene Wertebereiche, beispielsweise bevorzugte und weiter bevorzugte Wertebereiche, in der vorliegenden Erfindung angegeben sind, sind die Untergrenzen und die Obergrenzen der verschiedenen Wertebereiche miteinander kombinierbar. Der Begriff "Tiefe" der ersten und/oder zweiten Schlauchführung beschreibt den Abstand von der Kante, an der die erste und/oder zweite Schlauchführung an die Mantelfläche des Kanülenrohres grenzt, zum tiefsten Punkt der ersten und/oder zweiten Schlauchführung beziehungsweise einem Nutengrund der ersten und/oder zweiten Schlauchführung.

Der Begriff "im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

Beispielhafte Aufzählungen sind im Sinne der Erfindung als nicht abschließend anzusehen, sondern können im Rahmen des allgemeinen Fachwissens ergänzt werden.

Unter den Begriffen "kontinuierlich" und "stetig" im Sinne der Erfindung ist eine Kontinuität beispielsweise einer Oberfläche zu verstehen, die bevorzugt ununterbrochen ist. Beispielsweise ist unter einer kontinuierlichen Abnahme der Wandstärke zu verstehen, dass die Wandstärke über die Längserstreckung des Kanülenrohrs, bevorzugt des trachealen Endabschnittes, immer weiter abnimmt. Insbesondere findet keine wesentliche Unterbrechung der Abnahme über die Längserstreckung statt. Insbesondere ist unter "kontinuierlich" und "stetig" zu verstehen, dass kein Versprung oder Versatz in einer Oberfläche oder in einem Querschnitt einer Oberfläche visuell und/oder taktil erkennbar ist. Beispielsweise ist im Wesentlichen keine Fuge oder Versprung zwischen der Mantelfläche des Kanülenrohrs und dem ersten und/oder zweiten Schlauch vorhanden. Beispielsweise umfasst die Wandung des trachealen Endabschnitts, der zum trachealen Ende hin kontinuierlich abnimmt, keinen Versatz oder Versprung, bevorzugt über seine vollständige Länge.

Unter dem Begriff "Querschnitt" ist eine Schnittansicht zu verstehen, die senkrecht zu einer Längserstreckung des geschnittenen Teiles ist. Bevorzugt ist ein Vektor der Ansichtsebene des Querschnittes parallel zum Vektor der Längserstreckung an der Schnittstelle.

Unter dem Begriff "bündig" wird im Sinne der Erfindung verstanden, dass zwei Teile unmittelbar aneinandergrenzen und sich deren Oberflächen im Wesentlichen ergänzen. Beispielsweise kann der Schlauch bündig mit der Mantelfläche des Kanülenrohrs ausgestaltet sein, wobei zumindest ein Abschnitt der Oberfläche des Schlauches und ein Abschnitt der Mantelfläche ohne Versprung zueinander angeordnet sind. Bevorzugt haben bündig angeordnete Teile den Anschein, dass ihre Oberflächen sich zumindest abschnittsweise ergänzen.

In einer Ausgestaltung ist vorgesehen, dass das Kanülenrohr einen trachealen Endabschnitt und einen distalen Endabschnitt umfasst. Der distale Endabschnitt ist derart ausgebildet, dass dieser eine superiore Fläche und einen dieser gegenüberliegend angeordnete, inferiore Fläche aufweist. Das Kanülenschild weist einen Kanülenrohrausschnitt auf, in den der distale Endabschnitt des Kanülenrohrs anordenbar ist. Das Kanülenschild weist einen gradlinigen, den Kanülenrohrausschnitt begrenzenden ersten Abschnitt auf, welcher der superioren Fläche des distalen Endabschnittes zugeordnet ist, und einen gradlinigen, den Kanülenrohrausschnitt begrenzenden, zweiten Abschnitt aufweist, welcher der inferioren Fläche des distalen Endabschnittes zugeordnet ist. Das Kanülenschild ist mittels eines Drehlagers am distalen Endabschnitt um eine medial-laterale Achse kippbar angeordnet.

Eine medial-laterale Achse ist definiert als diejenige Achse, die bei bestimmungsgemäßen Gebrauch der Trachealkanüle im Wesentlichen senkrecht auf der Medianebene beziehungsweise Spiegelebene der Körpersymmetrie eines Benutzers steht. Bevorzugt erstreckt sich die medial-laterale Achse von medial nach lateral.

Die Trachealkanüle umfasst ein Kanülenrohr. Das Kanülenrohr umfasst ein distales erstes Ende und ein proximales zweites Ende.

Werden im Rahmen der Beschreibung der Erfindung Richtungsangaben verwendet, so sind diese in Bezug auf den üblichen Gebrauch der Trachealkanüle zu verstehen. Richtungsbezeichnungen werden wie in der Anatomie üblich verwendet. Unter dem Begriff "distal" wird im Sinne der vorliegenden Erfindung in Bezug auf ein Merkmal der erfindungsgemäßen Vorrichtung eine Anordnung beziehungsweise nicht beanspruchte Verwendung desselben fern oder auch abgewandt oder gegenüberliegend der Trachealkanüle, allgemeiner einer Hautoberfläche einer Person, die insbesondere die erfindungsgemäße Vorrichtung trägt, verstanden. Unter distal ist vorzugsweise eine Richtung vom Körper des Patienten weg zu verstehen. Unter dem Begriff "proximal" wird im Sinne der vorliegenden Erfindung in Bezug auf ein Merkmal der erfindungsgemäßen Vorrichtung eine Anordnung oder nicht beanspruchte Verwendung desselben nah oder auch zugewandt einer Hautoberfläche einer Person, die insbesondere solche Befestigungsmittel für die erfindungsgemäße Vorrichtung trägt, verstanden. Unter proximal ist vorzugsweise eine Richtung zum Körper des Patienten hin zu verstehen. Unter "lateral" ist vorzugsweise eine Richtung seitlich nach rechts und/oder links von einer Medianebene beziehungsweise einer Medianlinie aus zu verstehen. Unter "superior" ist vorzugsweise eine relative Richtungsangabe längs oder parallel der Körperlängsachse oberhalb eines Bezuges zu verstehen. Unter "inferior" ist vorzugsweise eine relative Richtungsangabe eine Richtung längs oder parallel der Körperlängsachse unterhalb eines Bezuges zu verstehen. Unter "tracheal" ist eine relative Richtungsangabe zu verstehen, die bei bestimmungsgemäßen Gebrauch der Trachea zugewandt ist.

Das Kanülenrohr umfasst eine Kanülenausnehmung, die insbesondere vom distalen ersten Ende bis zum trachealen zweiten Ende durchgängig ist. Insbesondere umfasst die Kanülenausnehmung einen Innendurchmesser. In einer Ausgestaltung ist vorgesehen, dass die vom distalen Ende zum trachealen Ende durchgehende Kanülenausnehmung bevorzugt über eine vollständige Längserstreckung, weiter bevorzugt über eine Längserstreckung des trachealen Endabschnittes einen gleichbleibenden Innendurchmesser umfasst.

Im Sinne der Erfindung ist unter einem gleichbleibenden Innendurchmesser zu verstehen, dass der Innendurchmesser im Wesentlichen über die vollständige Längserstreckung der Trachealkanüle oder zumindest des trachealen Endabschnittes, etwa gleich ist. Toleranzabweichungen von etwa 0,5 mm bis etwa 2,5 mm, bevorzugt etwa 1 mm sind ebenfalls unter "gleichbleibend" zu verstehen.

Das Kanülenrohr umfasst bevorzugt eine Wandung. Die Wandung umschließt bevorzugt die Kanülenausnehmung. Bevorzugt umfasst die Wandung eine Mantelfläche. Die Mantelfläche ist bevorzugt die Außenfläche der Wandung des Kanülenrohrs. In einer Ausgestaltung ist die Mantelfläche respektive die Wandung gesiebt, gefenstert oder ungefenstert ausgestaltet. Bevorzugt weist die Mantelfläche in Längsrichtung des Kanülenrohrs keinen Versprung auf. Weiter bevorzugt umfasst die Mantelfläche in Längsrichtung des Kanülenrohrs einen kontinuierlichen Verlauf.

In einer Ausgestaltung ist vorgesehen, dass ein Außenumfang der Trachealkanüle in einem Querschnitt senkrecht zu einer Erstreckung des Kanülenrohrs zumindest abschnittsweise im Wesentlichen oval ist.

Bevorzugt umfasst die Wandung eine erste Schlauchführung. In einer Ausgestaltung ist vorgesehen, dass die Trachealkanüle zumindest die erste Schlauchführung umfasst, die sich bevorzugt von einer distalen Seite des Kanülenschilds, weiter bevorzugt einer distalen Seite des distalen Endabschnitts des Kanülenrohrs, zumindest bis zu einem Cuff erstreckt, das dem trachealen Ende zugeordnet ist. Bevorzugt erstreckt sich die Schlauchführung bis zum trachealen Ende des Kanülenrohrs. Bevorzugt ist die erste Schlauchführung zur Aufnahme eines ersten Schlauches ausgestaltet. In einer Ausgestaltung ist vorgesehen, dass die erste Schlauchführung lateral in der Wandung angeordnet ist. Bevorzugt ist die erste Schlauchführung als Ausnehmung in der Wandung ausgestaltet. Bevorzugt erstreckt sich die Schlauchausnehmung in einer Längsrichtung des Kanülenrohrs, bevorzugt des trachealen Endabschnitts. In einer Ausgestaltung ist vorgesehen, dass die zumindest eine erste Schlauchführung zumindest Abschnittsweise als Nut in der Mantelfläche der Wandung ausgebildet ist. Bevorzugt ist vorgesehen, dass die erste Schlauchführung eine gleichbleibende Tiefe über die Länge der Nut oder gleichbleibende Materialstärke von Nutengrund zu Innenwandung der Kanüle umfasst. In einer weiteren Ausgestaltung ist vorgesehen, dass die Tiefe von distal nach tracheal variiert. Bevorzugt wird die Tiefe von distal nach lateral bevorzugt stetig geringer.

In einer Ausgestaltung ist vorgesehen, dass die Wandung des Kanülenrohrs zumindest des trachealen Endabschnitts in einem Querschnitt senkrecht zur Längserstreckung des Kanülenrohrs zumindest einseitig lateral eine Materialanhäufung aufweist. Die Materialanhäufung verdickt das Kanülenrohr zumindest einseitig bevorzugt lateral. Bevorzugt umfasst das Kanülenrohr zumindest an dem trachealen Endabschnitt zumindest abschnittsweise in einem Querschnitt senkrecht zur Längserstreckung des Kanülenrohrs auf beiden lateralen Seiten eine Materialanhäufung. Bevorzugt ist im Bereich des trachealen Endes des Kanülenrohrs beziehungsweise am trachealen Ende des Kanülenrohrs keine Materialanhäufung angeordnet. Bevorzugt umfasst ein verdickter Bereich des Kanülenrohrs eine Materialanhäufung. Bevorzugt erstreckt sich ein verdickter Bereich der Wandung beziehungsweise die Materialanhäufung auf einer lateralen Seite des Kanülenrohrs, bevorzugt des trachealen Endabschnitts in Längsrichtung des Kanülenrohrs. In einer Ausgestaltung nimmt eine Wandstärke des verdickten Bereiches, beziehungsweise des Bereiches der Materialanhäufung, in Längsrichtung des Kanülenrohrs ab. Bevorzugt nimmt die Wandstärke des verdickten Bereichs, beziehungsweise des Bereiches der bevorzugt lateralen Materialanhäufung, in Längsrichtung des Kanülenrohrs kontinuierlich ab. Bevorzugt nimmt die Wandstärke des verdickten Bereichs, beziehungsweise des Bereiches der bevorzugt lateralen Materialanhäufung, in Längsrichtung des Kanülenrohrs im gleichen Maße ab, wie die Wandstärke des inferioren Bereichs und/oder superioren Bereichs. Bevorzugt nimmt die Wandstärke des verdickten Bereichs, beziehungsweise des Bereiches der bevorzugt lateralen Materialanhäufung, in Längsrichtung des Kanülenrohrs schneller ab, als die Wandstärke des inferioren Bereichs und/oder superioren Bereichs. In einer Ausgestaltung ist vorgesehen, dass zumindest die erste Schlauchführung in einem verdickten Bereich der Wandung beziehungsweise in der Materialanhäufung ausgebildet ist. Weiter bevorzugt ist vorgesehen, dass zumindest die zweite Schlauchführung in einem verdickten Bereich der Wandung beziehungsweise in der Materialanhäufung ausgebildet ist. Bevorzugt ist eine Schlauchführung in einer Materialanhäufung als eine Nut ausgebildet.

Die erste Schlauchführung ist in einem Querschnitt des Kanülenrohrs bevorzugt rechteckig, oval, linsenförmig, bogenförmig oder kreisrund. In einer Ausgestaltung gleicht die erste Schlauchführung in einen Querschnitt einem Oval-Segment. In einer Ausgestaltung ist die erste Schlauchführung in einem Querschnitt senkrecht zur Längserstreckung des Kanülenrohrs u-förmig ausgestaltet.

In einer Ausgestaltung ist vorgesehen, dass die erste Schlauchführung in einem Querschnitt senkrecht zur Längserstreckung des Kanülenrohrs bogenförmig oder kreissegmentförmig ausgestaltet ist. Bevorzugt ist der Querschnitt der ersten Schlauchführung einem Querschnitt eines ersten Schlauches angepasst. Bevorzugt ist die erste Schlauchführung zur Aufnahme des ersten Schlauches angepasst. Besonders bevorzugt formt oder ergänzt der in die erste Schlauchführung eingesetzte erste Schlauch die Mantelfläche. Bevorzugt formt oder ergänzt der in die erste Schlauchführung eingesetzte erste Schlauch die Mantelfläche derart, dass diese stetig ausgebildet ist. Besonders bevorzugt ist die erste Schlauchführung derart ausgebildet, dass der erste Schlauch in dieser bündig mit der Mantelfläche des Kanülenrohrs ausgebildet ist.

Bevorzugt umfasst die Wandung eine zweite Schlauchführung. Bevorzugt ist die zweite Schlauchführung zur Aufnahme eines zweiten Schlauches ausgestaltet. In einer Ausgestaltung ist vorgesehen, dass die Trachealkanüle zumindest eine zweite Schlauchführung umfasst, die sich von einer distalen Seite des Kanülenschilds zumindest bis zum Cuff erstreckt und in der ein zweiter Schlauch geführt ist. In einer Ausgestaltung ist vorgesehen, dass die zweite Schlauchführung bis zum zweiten trachealen Ende des Kanülenrohrs erstreckt. In einer Ausgestaltung ist vorgesehen, dass die zweite Schlauchführung lateral in der Wandung angeordnet ist. Bevorzugt ist die zweite Schlauchführung auf lateral gegenüberliegend der ersten Schlauchführung angeordnet. Bevorzugt ist die zweite Schlauchführung als Ausnehmung in der Wandung ausgestaltet. In einer Ausgestaltung ist vorgesehen, dass die zweite Schlauchführung rohrförmig ausgestaltet ist. In einer Ausgestaltung ist vorgesehen, dass die zumindest die zweite Schlauchführung zumindest Abschnittsweise als Nut in der Mantelfläche der Wandung ausgebildet ist. Bevorzugt ist vorgesehen, dass die zweite Schlauchführung eine gleichbleibende Nut-Tiefe über die Länge der Nut oder gleichbleibende Materialstärke von Nutengrund zu Innenwandung der Kanüle umfasst. In einer weiteren Ausgestaltung ist vorgesehen, dass die Nut-Tiefe von distal nach tracheal variiert. Bevorzugt wird die Nut-Tiefe von distal nach tracheal bevorzugt stetig geringer. Bevorzugt wird die Nut-Tiefe von distal nach tracheal stetig geringer durch die bevorzugt kontinuierliche Abnahme der Wandstärke des verdickten Bereichs, beziehungsweise des Bereiches der bevorzugt lateralen Materialanhäufung, in Längsrichtung des Kanülenrohrs.

Die zweite Schlauchführung ist in einem Querschnitt des Kanülenrohrs bevorzugt rechteckig, oval, linsenförmig, bogenförmig oder kreisrund. In einer Ausgestaltung gleicht die zweite Schlauchführung in einen Querschnitt einem Oval-Segment. In einer Ausgestaltung ist die zweite Schlauchführung in einem Querschnitt senkrecht zur Längserstreckung des Kanülenrohrs u-förmig ausgestaltet. In einer Ausgestaltung ist vorgesehen, dass die erste Schlauchführung in einem Querschnitt senkrecht zur Längserstreckung des Kanülenrohrs bogenförmig oder kreissegmentförmig ausgestaltet ist. Bevorzugt ist der Querschnitt der zweiten Schlauchführung einem Querschnitt eines zweiten Schlauches angepasst. Bevorzugt ist die zweite Schlauchführung zur Aufnahme des zweiten Schlauches angepasst. Besonders bevorzugt formt oder ergänzt der in die zweite Schlauchführung eingesetzte zweite Schlauch die Mantelfläche. Bevorzugt formt oder ergänzt der in die zweite Schlauchführung eingesetzte zweite Schlauch die Mantelfläche derart, dass diese im Wesentlichen stetig ausgebildet ist. Besonders bevorzugt ist die zweite Schlauchführung derart ausgebildet, dass der zweite Schlauch in dieser bündig mit der Mantelfläche des Kanülenrohrs ausgebildet ist.

Die erste und/oder die zweite Schlauchführung sind in einer Ausführung derart ausgestaltet, dass diese durch eine Verjüngung des Kanülenrohrs über die Länge des Kanülenrohrs zum trachealen Ende hin flacher wird. In einer Ausführungsform ist vorgesehen, dass der erste und/oder zweite Schlauch über seine Länge oder über einen Abschnitt etwa den gleichen Außendurchmesser, Innendurchmesser und/oder den gleichen Querschnitt aufweist. In einer Ausführungsform ist vorgesehen, dass der erste und/oder zweite Schlauch über seine Länge den Außendurchmesser, den Innendurchmesser und/oder den Querschnitt ändert. Bevorzugt ist der Außendurchmesser, der Innendurchmesser und/oder der Querschnitt des ersten und/oder zweiten Schlauchs über seine Länge der ersten und/oder zweien Schlauchführung angepasst. In einer Ausführungsform ist vorgesehen, dass der erste und/oder zweite Schlauch in einem Abschnitt angrenzend an den distalen Endabschnitt im Wesentlichen vollständig von der ersten und/oder zweiten Schlauchausnehmung im trachealen Endabschnitt aufgenommen ist beziehungsweise mit der Mantelfläche im Wesentlichen bündig abschließt. In einer Ausführungsform ist vorgesehen, dass der erste und/oder zweite Schlauch im trachealen Endabschnitt im Wesentlichen vollständig von der ersten und/oder zweiten Schlauchausnehmung über deren gesamte Länge aufgenommen ist beziehungsweise mit der Mantelfläche im Wesentlichen bündig abschließt. Vorteilhaft an der vollständigen Aufnahme des ersten und/oder zweiten Schlauches über die gesamte Länge der Schlauchausnehmung im trachealen Endabschnitt ist, dass eine Traumatisierung des Gewebes beim Einführen und Herausziehen der Trachealkanüle in und aus der Trachea vermieden wird. In einer Ausführungsform ist vorgesehen, dass der erste und/oder zweite Schlauch in Erstreckungsrichtung der ersten und/oder zweiten Schlauchführung in Richtung des trachealen Endes des Kanülenrohrs immer weiter über eine Mantelfläche des Kanülenrohrs hinaus ragt. In einer Ausführungsform gibt es einen ersten und/oder einen zweiten Schlauch, die jeweils in der ersten und/oder zweiten Schlauchführung des Kanülenrohrs angeordnet sind. Die Tiefe der Schlauchführung und/oder der Durchmesser der Schläuche in radialer Richtung des Kanülenrohrs können über ihre jeweilige Länge variieren. Der erste und/oder der zweite Schlauch ragt in Erstreckungsrichtung bevorzugt radial immer weiter aus der Schlauchführung heraus. In Richtung des trachealen Endes erhebt sich der der erste und/oder zweite Schlauch bevorzugt weiter aus der Schlauchführung beziehungsweise über die Mantelfläche des Kanülenrohrs als am distalen Ende. Bevorzugt erhebt sich der erste und/oder zweite Schlauch über seine Länge stetig über die Mantelfläche des Kanülenrohrs. Vorteilhaft wird durch die stetige Erhebung des Schlauches über die Mantelfläche eine Traumatisierung des Gewebes beim Herauziehen der Trachealkanüle aus der Trachea vermieden.

In einer Ausgestaltung ist vorgesehen, dass zumindest die erste Schlauchführung und/oder die zweite Schlauchführung in einem Bereich der Materialanhäufung der Wandung ausgebildet ist. In einer Ausgestaltung ist zumindest eine, bevorzugt sind beide Schlauchführungen in einem Bereich der Trachealkanüle angeordnet, die eine Materialanhäufung aufweist. Bevorzugt umfasst die Trachealkanüle zwei bevorzugt etwa gegenüberliegende Materialanhäufungen, die jeweils eine Schlauchführung umfassen. Bevorzugt umfasst die zumindest eine Materialanhäufung eine Nut, die als erste oder zweite Schlauchführung fungiert. Die zumindest eine Materialanhäufung erstreckt sich bevorzugt in Längsrichtung der Trachealkanüle. Im Bereich des trachealen Endes der ersten und/oder zweiten Schlauchführung weist die Trachealkanüle bevorzugt jeweils eine Materialanhäufung auf, die wesentlich über die Wandstärke des umgebenen Bereiches herausragt. Eine Wandstärke zwischen dem trachealen Ende und einem Ende der ersten und zweiten Schlauchführung ist bevorzugt dünner als die Wandstärke im Bereich der Schlauchführung ausgestaltet und weist eine geringere oder keine Materialanhäufung auf. Die Wandstärke nimmt bei der Trachealkanüle bevorzugt zum distalen Ende hin stetig zu. Die Wandstärke im Bereich der Materialanhäufung in Richtung der Längserstreckung der Trachealkanüle bleibt bevorzugt über die Länge der Schlauchführung gleich. In einer weiteren Ausführungsform vergrößert sich die Wandstärke im Bereich der Materialanhäufung in Richtung der Längserstreckung der Trachealkanüle nach distal zumindest über die Länge der Schlauchführung. Vorteilhaft können die erste und/oder zweite Schlauchführungen über die Längserstreckung der Trachealkanüle gleich tief in der Materialanhäufung eingebettet sein.

In einer Ausgestaltung ist vorgesehen, dass die Wandung des Kanülenrohrs einen ersten Außendurchmesser aufweist. Bevorzugt weist ein Abschnitt der Wandung, bevorzugt ein Abschnitt des Kanülenrohrs, weiter bevorzugt der tracheale Endabschnitt des Kanülenrohrs, den ersten Außendurchmesser auf. Bevorzugt weist das Kanülenrohr, weiter bevorzugt der tracheale Endabschnitt des Kanülenrohrs an einem dem Kanülenschild zugewandten Ende den ersten Außendurchmesser auf. Bevorzugt ist der erste Außendurchmesser der maximale Außendurchmesser des Kanülenrohrs, bevorzugt der maximale Außendurchmesser des trachealen Endabschnittes des Kanülenrohrs.

In einer weiteren Ausgestaltung umfasst das Kanülenrohr einen zweiten Außendurchmesser. Bevorzugt umfasst ein Abschnitt der Wandung, bevorzugt ein Abschnitt des Kanülenrohrs, weiter bevorzugt der tracheale Endabschnitt des Kanülenrohrs, den zweiten Außendurchmesser. Bevorzugt weist das Kanülenrohr, weiter bevorzugt der tracheale Endabschnitt des Kanülenrohrs im Bereich des trachealen Endes, bevorzugt am trachealen Ende, weiter bevorzugt an einem Cuff den zweiten Außendurchmesser auf.

Bevorzugt umfasst die Trachealkanüle eine vom distalen Ende zum trachealen Ende durchgehende Kanülenausnehmung mit einem gleichbleibenden Innendurchmesser. Weiter bevorzugt ist der erste Außendurchmesser größer als der zweite Außendurchmesser. Weiter bevorzugt ist eine Mantelfläche der Wandung konisch ausgebildet. In einer Ausgestaltung ist vorgesehen, dass die Wandung des Kanülenrohrs zumindest in einem trachealen Endabschnitt sich zu einem trachealen Ende hin verjüngt. In einer Ausgestaltung ist vorgesehen, dass die Wandung einen ersten Außendurchmesser, der dem Kanülenschild zugeordnet ist, und einen zweiten Außendurchmesser, der dem Cuff beziehungsweise dem trachealen Ende zugeordnet ist, umfasst, wobei der erste Außendurchmesser größer ist als der zweite Außendurchmesser. In einer weiteren Ausgestaltung ist vorgesehen, dass eine Wandung zumindest des tracheale Endabschnitts eine erste Wandstärke angrenzend an den distalen Endabschnitt und eine zweite Wandstärke angrenzend an das trachealen Endes umfasst, wobei die erste Wandstärke größer ist als die zweite Wandstärke ist. In einer weiteren Ausgestaltung ist vorgesehen, dass eine Wandstärke des trachealen Endabschnitts zum trachealen Ende hin kontinuierlich oder diskontinuierlich abnimmt.

In einer Ausgestaltung ist der zweite Außendurchmesser der kleinste Außendurchmesser der Trachealkanüle. Weiter bevorzugt ist der zweite Außendurchmesser, derjenige Außendurchmesser der Trachealkanüle, der von einem Operateur beim Einsetzen der Trachealkanüle in Abhängigkeit von der Größe des Tracheostoma ausgewählt wird. Bevorzugt ist der erste Außendurchmesser etwa 1 mm, bevorzugt genau 1 mm größer als eine Normgrößenbezeichnung der Trachealkanüle, die eine Nennweite, das heißt ein Innendurchmesser der Trachealkanüle bezeichnet. Vorteilhaft braucht ein Operateur einen Außendurchmesser der Trachealkanüle nicht auszumessen, sondern kann eine vorgeschlagene Trachealkanüle anhand der Normgrößenbezeichnung für das jeweils geöffnete Tracheostoma auswählen. Hierbei braucht er lediglich zu berücksichtigen, dass der erste Außendurchmesser der Trachealkanüle etwa 1 mm größer ist als die Normgrößenbezeichnung.

In einer Ausgestaltung ist vorgesehen, dass der dem trachealen Ende zugeordnete tracheale Endabschnitt der Wandung dünner ist als der dem Kanülenschild zugeordnete distale Endabschnitt der Wandung. In einer Ausgestaltung ist vorgesehen, dass eine erste Wandstärke der Wandung an der Stelle des ersten Außendurchmessers des Kanülenrohrs größer ist als eine zweite Wandstärke der Wandung an der Stelle des zweiten Außendurchmessers des Kanülenrohrs, wobei der zweite Außendurchmesser dem trachealen Ende näher ist als der erste Außendurchmesser. Bevorzugt ist der tracheale Endabschnitt nach tracheal, also zum trachealen Ende hin, verjüngend ausgestaltet. Weiter bevorzugt ist ein dem Kanülenschild zugeordneter Abschnitt des trachealen Endabschnitts dickwandiger ausgestaltet als ein dem trachealen Ende zugeordneter Abschnitt des trachealen Endabschnitts. In einer Ausgestaltung ist eine Wandungsstärke der Wandung am trachealen Endabschnitt beziehungsweise am zweiten Außendurchmesser etwa 0,4 mm bis etwa 0,6 mm, bevorzugt etwa 0,5 mm.

Vorteilhaft steht der erste und/oder zweite Schlauch zumindest abschnittsweise nicht über einen ersten und/oder zweiten Außendurchmesser der Mantelfläche des Kanülenrohrs hervor.

In einer Ausgestaltung ist vorgesehen, dass die Trachealkanüle zumindest ein Material umfasst ausgewählt aus einer Gruppe zumindest umfassend Silikon, thermoplastische Elastomere, Polyvinylchlorid und/oder Polycarbonat.

In einer Ausgestaltung umfasst das Kanülenrohr den trachealen Endabschnitt und einen distalen Endabschnitt. Der distale Endabschnitt ist bevorzugt dem Kanülenschild zugeordnet. Weiter bevorzugt ist der distale Endabschnitt außerhalb der Trachea anordenbar. Weiter bevorzugt ist der tracheale Endabschnitt in der Trachea anordenbar. Weiter bevorzugt unterscheiden sich distaler Endabschnitt und trachealer Endabschnitt geometrisch voneinander. In einer Ausgestaltung ist vorgesehen, dass der distale Endabschnitt im Wesentlichen kastenförmig beziehungsweise kubisch ausgestaltet ist. Beispielsweise ist der distale Endabschnitt im Wesentlichen kastenförmig ausgestaltet, wobei der tracheale Endabschnitt im Wesentlichen rohrförmig ausgestaltet ist. Weiter bevorzugt ist vorgesehen, dass der distale Endabschnitt an einer distalen Seite des trachealen Endabschnittes angrenzt. In einer Ausführungsform ist der distale Endabschnitt zweiteilig ausgestaltet, wobei ein erster Teil materialeinheitlich mit dem trachealen Endabschnitt verbunden ist. Der zweite Teil ist bevorzugt formschlüssig und/oder materialschlüssig mit dem ersten Teil verbunden.

In einer Ausgestaltung umfasst der distale Endabschnitt eine Ausprägung von proximal nach distal. In einer weiteren Ausgestaltung umfasst der distale Endabschnitt eine laterale Ausprägung, bevorzugt größer als eine laterale Ausprägung des trachealen Endabschnittes. In einer weiteren Ausgestaltung umfasst der distale Endabschnitt eine Ausprägung von inferior nach superior, bevorzugt größer als eine Ausprägung des trachealen Endabschnitts von inferior noch superior. In einer Ausgestaltung ist vorgesehen, dass eine Erstreckung des distalen Endabschnittes von superior nach inferior im Wesentlichen einer Erstreckung des trachealen Endabschnittes an einer Kontaktstelle mit dem distalen Endabschnitt von superior nach inferior entspricht. In einer Ausgestaltung ist vorgesehen, dass der distale Endabschnitt des Kanülenrohrs lateral, inferior und/oder superior über einen ersten Außendurchmesser des trachealen Endabschnittes hinaus erstreckt.

Bevorzugt umfasst der distale Endabschnitt eine superiore Fläche, die bevorzugt durch eine Kante begrenzt ist. Bevorzugt ist die superiore Fläche im Wesentlichen eben ausgestaltet. Weiter bevorzugt ist die superiore Fläche im Wesentlichen rechteckig, insbesondere in einer Aufsicht von superior, ausgestaltet.

Bevorzugt umfasst der distale Endabschnitt eine inferiore Fläche, die bevorzugt durch eine Kante begrenzt ist. Bevorzugt ist die inferiore Fläche im Wesentlichen eben ausgestaltet. Weiter bevorzugt ist die inferiore Fläche im Wesentlichen rechteckig, insbesondere in einer Ansicht von inferior, ausgestaltet. Bevorzugt ist die inferiore Fläche im Wesentlichen parallel zur superioren Fläche.

Bevorzugt umfasst der distale Endabschnitt laterale Flächen. Bevorzugt sind die lateralen Flächen eben, gewölbt, gerundet oder nach lateral konvex ausgestaltet. Bevorzugt sind die lateralen Flächen, bevorzugt in einer Ansicht von lateral, rechteckig. Die lateralen Flächen sind bevorzugt durch die superiore Fläche und die inferiore Fläche sowie durch proximale und distale Flächen begrenzt.

Bevorzugt umfasst der distale Endabschnitt eine distale Fläche. Die distale Fläche ist bei Nutzung der Trachealkanüle bevorzugt im Wesentlichen parallel zu einer Frontalebene des Benutzers. Die distale Fläche ist bevorzugt durch die lateralen Flächen sowie die superiore und inferiore Fläche begrenzt. Bevorzugt ist die distale Fläche eben ausgestaltet. Weiter bevorzugt umfasst die distale Fläche zumindest eine Öffnung. In einer Ausgestaltung ist vorgesehen, dass zumindest der distale Endabschnitt eine Öffnung aufweist, die in den trachealen Endabschnitt mündet. Bevorzugt mündet die Öffnung in die Kanülenausnehmung.

In einer Ausgestaltung ist vorgesehen, dass der distale Endabschnitt sich ausgehend von einem Rand der Öffnung um etwa 1 mm bis etwa 5 mm, bevorzugt etwa 1 mm bis etwa 2 mm nach superior und/oder inferior erstreckt.

In einer Ausgestaltung ist vorgesehen, dass der distale Endabschnitt mindestens eine erste Schlauchausnehmung für den ersten Schlauch aufweist. Die Schlauchausnehmung ist bevorzugt in einer Ansicht von distal rund, linsenförmig oder oval ausgestaltet. Bevorzugt ist die erste Schlauchausnehmung an einen Querschnitt des ersten Schlauches angepasst. Bevorzugt umfasst die Schlauchausnehmung einen linsenförmigen, runden oder ovalen Querschnitt. Unter "linsenförmig" im Sinne der Erfindung ist eine Form zu verstehen, die einseitig oder beidseitig konvex ausgestaltet sein ist. Insbesondere kann die Form einseitig eben sein. Weiter bevorzugt können die beiden Hälften der Linsenform unterschiedliche Radien aufweisen. In einer Ausgestaltung ist die Linsenform im Querschnitt aus zwei Kreissegmenten zusammengesetzt. Bevorzugt umfasst die Linsenform oder die Ovalform eine Linsenachse und zwei Extremstellen, die durch die Kontaktstellen der Radien der einzelnen Linsenhälften beziehungsweise Ovalhälften definiert ist. Bevorzugt ist die Linsenachse bei Gebrauch der Trachealkanüle im Wesentlichen parallel zur Medianebene des Benutzers.

Bevorzugt ist die erste Schlauchausnehmung paramedian, das heißt deutlich außerhalb der Mittelachse der Trachealkanüle angeordnet. Weiter bevorzugt ist die erste Schlauchausnehmung lateral eines Anschlussmittels zur Verbindung mit einer Innenkanüle angeordnet. Vorteilhaft ist die erste Schlauchausnehmung somit weit genug außen gelegen, um den Anschluss einer Innenkanüle nicht zu behindern. Weiterhin vorteilhaft ist die Schlauchausnehmung im Wesentlichen in einer Flucht mit der ersten Schlauchführung des trachealen Endabschnittes. Weiter bevorzugt ist die erste Schlauchausnehmung auf einer Höhe oder inferior des lateralen Durchmessers der Öffnung angeordnet. Bevorzugt ist die erste Schlauchausnehmung etwa auf halber Höhe zwischen lateralen Durchmesser der Öffnung und einer inferioren Kante der distalen Fläche angeordnet. Vorteilhaft ist derart eine ausreichend großer Krümmungsradius der ersten Schlauchführung beziehungsweise des ersten Schlauches erreicht, der einen Durchfluss nicht Wesentlich behindert.

Bevorzugt erstreckt sich die erste Schlauchausnehmung in die beziehungsweise angrenzend bis zur ersten Schlauchführung. Weiter bevorzugt erstreckt sich die erste Schlauchausnehmung gerade oder gebogen durch den distalen Endabschnitt.

In einer Ausgestaltung ist vorgesehen, dass der distale Endabschnitt mindestens eine zweite Schlauchausnehmung für den zweiten Schlauch aufweist. Die Schlauchausnehmung ist bevorzugt in einer Ansicht von distal rund, linsenförmig oder oval ausgestaltet. Bevorzugt ist die zweite Schlauchausnehmung an einen Querschnitt des zweiten Schlauches angepasst. Bevorzugt umfasst die Schlauchausnehmung einen linsenförmigen, runden oder ovalen Querschnitt. Bevorzugt ist eine Linsensachse der zweiten Schlauchausnehmung bei Gebrauch der Trachealkanüle im Wesentlichen parallel zur Medianebene des Benutzers.

Bevorzugt ist die zweite Schlauchausnehmung in Relation zu einer Medianebene des distalen Endabschnittes gegenüberliegend der ersten Schlauchausnehmung angeordnet. Bevorzugt ist die zweite Schlauchausnehmung paramedian, das heißt deutlich außerhalb der Mittelachse der Trachealkanüle angeordnet. Weiter bevorzugt ist die zweite Schlauchausnehmung lateral eines Anschlussmittels zur Verbindung mit einer Innenkanüle angeordnet. Vorteilhaft ist die zweite Schlauchausnehmung somit weit genug außen gelegen, um den Anschluss einer Innenkanüle nicht zu behindern. Weiterhin vorteilhaft ist die Schlauchausnehmung im Wesentlichen in einer Flucht mit der zweiten Schlauchführung des trachealen Endabschnittes. Weiter bevorzugt ist die zweite Schlauchausnehmung auf einer Höhe oder inferior des lateralen Durchmessers der Öffnung angeordnet. Bevorzugt ist die zweite Schlauchausnehmung etwa auf halber Höhe zwischen lateralen Durchmesser der Öffnung und einer inferioren Kante der distalen Fläche angeordnet. Vorteilhaft ist derart eine ausreichend großer Krümmungsradius der zweiten Schlauchführung beziehungsweise des zweiten Schlauches erreicht, der einen Durchfluss nicht Wesentlich behindert.

Bevorzugt erstreckt sich die zweite Schlauchausnehmung in die zweite Schlauchführung beziehungsweise grenzt an die zweiten Schlauchführung an. Weiter bevorzugt erstreckt sich die zweite Schlauchausnehmung gerade oder gebogen durch den distalen Endabschnitt.

In einer weiteren Ausgestaltung ist vorgesehen, dass der distale Endabschnitt ein Anschlussmittel umfasst. Bevorzugt ist das Anschlussmittel zur Verbindung der Trachealkanüle mit einer Innenkanüle ausgestaltet. Bevorzugt ist das Anschlussmittel der Öffnung zugeordnet. Weiter bevorzugt ist das Anschlussmittel zumindest teilweise umlaufend um die Öffnung angeordnet. Bevorzugt ist das Anschlussmittel ein Bajonettverschlussteil, das weiter bevorzugt mit einem Bajonettverschlussteil einer Innenkanüle zusammen wirken kann. Bevorzugt ist das Anschlussmittel auf der distalen Fläche angeordnet. Weiter bevorzugt erstreckt sich das Anschlussmittel von der distalen Fläche nach distal.

Weiterhin wird ein Schlauch zur Verwendung mit einer beschriebenen Trachealkanüle vorgeschlagen. In einer Ausgestaltung ist vorgesehen, dass der Schlauch in einem Querschnitt senkrecht zur Längserstreckung des ersten Schlauches oval, linsenförmig oder kreisrund ausgestaltet ist. Bevorzugt umfasst der Schlauch ein thermoplastisches Material.

In einer bevorzugten Ausgestaltung ist der Schlauch oval oder linsenförmig ausgestaltet. Bevorzugt ist der Schlauch aus einem runden Schlauch gefertigt, welcher bevorzugt der Länge nach aufgetrennt und nach einer bevorzugt thermischen Umformung wieder zusammen gefügt ist. In einer bevorzugten Ausgestaltung des ersten Schlauches und/oder des zweiten Schlauches ist dieser in einem Querschnitt linsenförmig ausgestaltet. Bevorzugt ist der Querschnitt des Schlauches, besonders bevorzugt der Querschnitt der Mantelfläche des Schlauches, linsenförmig ausgestaltet, weiter bevorzugt beidseitig konvex ausgebildet.

Weiterhin wird ein Schlauch, insbesondere geeignet zur Verwendung mit einer Trachealkanüle, umfassend zwei sich in Längserstreckung des ersten und/oder zweiten Schlauches erstreckende, im Querschnitt senkrecht zur Längserstreckung konvexe Schlauchabschnitte vorgeschlagen, wobei ein erster Schlauchabschnitt im Querschnitt einen ersten Radius R1 umfasst und ein zweiter Schlauchabschnitt im Querschnitt einen zweiten Radius R2 umfasst, wobei der erste Radius R1 kleiner ist als der zweite Radius R2 ist.

In einer Ausgestaltung ist der Schlauch der erste und/oder der zweite Schlauch, der oben beschrieben ist.

In einer Ausgestaltung weist der Schlauch zwei Schlauchabschnitte auf. Die Schlauchabschnitte sind bevorzugt sich in Längserstreckung des Schlauches erstreckende, im Querschnitt beziehungsweise in einer Aufsicht auf eine Stirnseite konvexe Abschnitte. Bevorzugt sind die beiden Schlauchabschnitte der Länge nach verbunden, bevorzugt materialverbunden, weiter bevorzugt verschweißt. In einer Ausgestaltung sind die konvexen Schlauchabschnitte unterschiedlich geformt. Der erste Schlauchabschnitt umfasst bevorzugt im Querschnitt einen ersten Radius R1. Der zweite Schlauchabschnitt umfasst bevorzugt im Querschnitt einen zweiten Radius R2. Bevorzugt ist der erste Radius R1 kleiner ist als der zweite Radius R2. Bevorzugt ist der erste Schlauchabschnitt dem Nutengrund der jeweiligen Schlauchausnehmung zugeordnet. In einer Ausgestaltung hat der Außenumfang des ersten und/oder zweiten Schlauchabschnittes etwa den gleichen Radius wie der Außenumfang des Kanülenrohrs, bevorzugt an der Grenze zum distalen Endabschnitt. In einer Ausgestaltung hat der Außenumfang des ersten und/oder zweiten Schlauchabschnittes etwa den gleichen Radius wie eine Materialanhäufung des Kanülenrohrs, bevorzugt etwa den gleichen Radius wie eine Materialanhäufung, für dessen Schlauchausnehmung der Schlauch vorgesehen ist. In einer Ausgestaltung ist vorgesehen, dass der Schlauch aus einem runden Schlauch gefertigt ist, welcher bevorzugt der Länge nach aufgetrennt und nach einer bevorzugt thermischen Umformung wieder zusammen gefügt ist.

In einer Ausgestaltung umfasst die Trachealkanüle einen ersten Schlauch. Bevorzugt kann der erste Schlauch als Absaugschlauch verwendet werden. In einer Ausgestaltung ist vorgesehen, dass der erste Schlauch ein Absaugschlauch ist, der superior des Cuffs endet. In einer Ausgestaltung ist vorgesehen, dass der erste Schlauch zumindest teilweise in der ersten Schlauchführung angeordnet ist. Bevorzugt erstreckt sich der erste Schlauch von distal durch die erste Schlauchausnehmung in die erste Schlauchführung. Weiter bevorzugt erstreckt sich der erste Schlauch vom distalen Endabschnitt nach distal und weist weiter bevorzugt einen Konnektor für eine Absaugvorrichtung auf. In einer Ausgestaltung ist vorgesehen, dass der erste Schlauch im Querschnitt oval, linsenförmig oder kreisrund ausgestaltet ist. Bevorzugt ist der erste Schlauch im Querschnitt linsenförmig ausgestaltet. Bevorzugt ist der erste Schlauch derart an die erste Schlauchführung angepasst, dass zwischen der Mantelfläche des trachealen Endabschnitts und dem ersten Schlauch im Wesentlichen keine Fuge ist. Vorteilhaft wird hierdurch ein atraumatisches, das heißt gewebeschonendes, Einführen der Trachealkanüle und eine komfortable Nutzung erreicht. Bevorzugt ist der erste Schlauch derart in der ersten Schlauchführung angeordnet, dass eine aus der ersten Schlauchführung herausragende Teiloberfläche des ersten Schlauches eine Umhüllende der Mantelfläche ergänzt. In einer Ausgestaltung ist vorgesehen, dass die Umhüllende, welche die Mantelfläche und den ersten Schlauch in der ersten Schlauchführung umhüllt, in einem Querschnitt im Wesentlichen kreisrund oder oval ist. In einer Ausgestaltung ist vorgesehen, dass eine Umhüllende, welche eine Mantelfläche der Wandung und den ersten Schlauch in der ersten Schlauchführung umhüllt, in einem Querschnitt im Wesentlichen stetig ist. In einer Ausgestaltung ist vorgesehen, dass eine Umhüllende, welche eine Mantelfläche der Wandung und den zweiten Schlauch in der zweiten Schlauchführung umhüllt, in einem Querschnitt im Wesentlichen stetig ist. In einer Ausführungsform ist vorgesehen, dass ein Außenumfang des ersten Schlauchs über eine Kreisform oder eine Ovalform der Mantelfläche hinausragt beziehungsweise eine Erhebung in der einer Kreisform oder eine Ovalform der Umhüllenden bildet. Insbesondere ragt der Außenumfang des ersten Schlauches um etwa 0,01 mm bis etwa 1 mm über eine Kreisform oder eine Ovalform hinaus. Bevorzugt bilden erster Schlauch und Mantelfläche eine stetige Oberfläche aus.

In einer Ausgestaltung ist vorgesehen, dass der Außendurchmesser des ersten Schlauches in radialer Erstreckung des Kanülenrohrs kleiner oder etwa gleich einer Tiefe der ersten Schlauchführung ist.

In einer Ausgestaltung umfasst die Trachealkanüle einen zweiten Schlauch. Bevorzugt kann der zweite Schlauch als Füllschlauch verwendet werden. In einer Ausgestaltung ist vorgesehen, dass der zweite Schlauch ein Füllschlauch für das Cuff ist, der sich in das Cuff hinein erstreckt. Bevorzugt ist mittels des zweiten Schlauchs Luft in das Cuff füllbar. In einer Ausgestaltung ist vorgesehen, dass der zweite Schlauch zumindest teilweise in der zweiten Schlauchführung angeordnet ist. Bevorzugt erstreckt sich der zweite Schlauch von distal durch die zweite Schlauchausnehmung in die zweite Schlauchführung. Weiter bevorzugt erstreckt sich der zweite Schlauch vom distalen Endabschnitt nach distal und weist weiter bevorzugt einen Konnektor für eine Absaugvorrichtung auf. In einer Ausgestaltung ist vorgesehen, dass der zweite Schlauch im Querschnitt oval, linsenförmig oder kreisrund ausgestaltet ist. Bevorzugt ist der zweite Schlauch in einem Querschnitt im Wesentlichen kreisrund ausgebildet. Bevorzugt ist der zweite Schlauch derart an die zweite Schlauchführung angepasst, dass zwischen der Mantelfläche des trachealen Endabschnitts und dem zweiten Schlauch im Wesentlichen keine Fuge ist. Vorteilhaft wird hierdurch ein atraumatisches Einführen der Trachealkanüle und eine komfortable Nutzung erreicht. Bevorzugt ist der zweite Schlauch derart in der zweiten Schlauchführung angeordnet, dass eine aus der zweiten Schlauchführung herausragende Teiloberfläche des zweiten Schlauches eine Umhüllende der Mantelfläche ergänzt. In einer Ausgestaltung ist vorgesehen, dass die Umhüllende, welche die Mantelfläche und den zweiten Schlauch in der zweiten Schlauchführung umhüllt, in einem Querschnitt im Wesentlichen kreisrund oder oval ist. In einer Ausführungsform ist vorgesehen, dass ein Außenumfang des zweiten Schlauchs über eine Kreisform oder eine Ovalform der Mantelfläche hinausragt beziehungsweise eine Erhebung in der einer Kreisform oder eine Ovalform der Umhüllenden bildet. Insbesondere ragt der Außenumfang des zweiten Schlauches um etwa 0,01 mm bis etwa 1 mm über eine Kreisform oder eine Ovalform hinaus. Bevorzugt bilden zweiter Schlauch und Mantelfläche eine stetige Oberfläche aus.

In einer Ausgestaltung ist vorgesehen, dass der Außendurchmesser des zweiten Schlauches in radialer Erstreckung des Kanülenrohrs kleiner oder etwa gleich einer Tiefe der ersten Schlauchführung ist.

In einer Ausgestaltung umfasst die Trachealkanüle ein Kanülenschild. Bevorzugt umfasst das Kanülenschild einen Kanülenrohrausschnitt. Weiter bevorzugt erstreckt sich der Kanülenrohrausschnitt vollständig von proximal nach distal durch das Kanülenschild. In einer Aufsicht von distal ist der Kanülenrohrausschnitt bevorzugt rechteckig. Weiter bevorzugt umfasst der Kanülenrohrausschnitt superior und/oder inferior eine gerade Kante, die weiter bevorzugt parallel zueinander angeordnet sind. Weiter bevorzugt umfasst der Kanülenrohrausschnitt gegenüberliegende laterale gerade Kanten, die bevorzugt parallel zueinander angeordnet sind. Weiter bevorzugt umfasst der Kanülenrohrausschnitt genüberliegende, gebogene laterale Kanten, die bevorzugt nach lateral konvex gebogen sind. Der Kanülenrohrausschnitt ist bevorzugt zumindest von einem ersten lateralen Abschnitt, einem zweiten lateralen Abschnitt und weiter bevorzugt von einer ersten superioren Abschnitt und einem ersten inferioren Abschnitt begrenzt. Der Kanülenrohrausschnitt ist bevorzugt zur Aufnahme des distalen Endabschnitts des Kanülenrohrs geeignet. Bevorzugt ist das Kanülenrohr mittels eines Drehlagers im Kanülenrohrausschnitt gelagert.

In einer Ausgestaltung ist vorgesehen, dass das Kanülenschild laterale, den Kanülenrohrausschnitt begrenzenden Abschnitte aufweist, wobei das Drehlager zwischen den lateralen Abschnitten des Kanülenrohrausschnitts und lateralen Flächen des Endabschnitts des Kanülenrohrs angeordnet ist. In einer Ausführungsform ist vorgesehen, dass die lateralen Abschnitte Lagerausnehmungen oder Lagerstifte aufweisen, die mit Lagerstiften oder Lagerausnehmungen zusammenwirken, die an dem distalen Endabschnitt angeordnet sind. Bevorzugt sind auf den lateralen Flächen des Endabschnitts Lagerstifte angeordnet oder Lagerausnehmungen eingebracht. Bevorzugt erstreckt sich eine Lagerachse, um die Kanülenschild und Kanülenrohr umeinander kippbar sind, in laterale Richtung. Weiter bevorzugt ist die Lagerachse in einer Aufsicht von distal mit der Achse des Durchmessers der Öffnung des Endabschnittes identisch. Vorteilhaft ist das Kanülenschild relativ zum Kanülenrohr kippbar, bevorzugt um die laterale Achse. Hierdurch wird eine komfortable Anpassung des Winkels von Kanülenschild, der auf einer Halsoberfläche anordenbar ist, und dem Kanülenrohr, das in der Trachea anordenbar ist, erreicht. Besonders vorteilhaft hat sich überraschend die Ausgestaltung der geraden posterioren und inferioren Kanten erwiesen. Durch diese Ausgestaltung ist das Kanülenschild zum Kanülenrohr in einem größeren Winkel kippbar, als dies aus dem Stand der Technik bekannt ist. In einer Ausgestaltung ist vorgesehen, dass durch eine Kippbewegung zumindest ein inferiorer und/oder superiorer Spalt zwischen dem distalen Endabschnitt des Kanülenrohrs und dem Kanülenschild zumindest teilweise geschlossen oder verringert wird. Bevorzugt wird ein Spalt zwischen den Wandungen des Kanülenrohrausschnitts und den Flächen des Endabschnitts verringert. Weiter bevorzugt ist die Kippbewegung begrenzt. Bevorzugt ist die Kippbewegung dadurch begrenzt, dass das Kanülenschild, bevorzugt ein posteriorer Abschnitt oder ein inferiorer Abschnitt des Kanülenrohrausschnitts gegen das Kanülenrohr stößt.

In einer Ausgestaltung umfasst die Trachealkanüle eine Innenkanüle. Die Innenkanüle ist gefenstert oder ungefenstert ausgestaltet. Bevorzugt ist die Innenkanüle mittels eines Bajonettverschlusses an der Trachealkanüle befestigbar.

In einer Ausgestaltung umfasst die Trachealkanüle ein Cuff. Bevorzugt ist das Cuff dem trachealen Endabschnitt zugeordnet. Weiter bevorzugt ragt der zweite Schlauch in das Cuff. Weiter bevorzugt ist das Cuff mittels des zweiten Schlauches aufblasbar und/oder kann mit diesem die Luft aus dem Cuff entzogen werden.

In einer beispielhaften Ausführungsform umfasst die Trachealkanüle einen ersten Schlauch für eine subglottische Absaugung und einen zweiten Schlauch zur Befüllung eines Cuffs. Das Kanülenrohr umfasst auf einer distalen Seite eine Öffnung um dessen Rand ein Anschlussmittel angeordnet ist, mittels dem eine Innenkanüle anschließbar ist. Weiterhin umfasst das Kanülenrohr beispielhaft eine Siebung im trachealen Endabschnitt. Ein distaler Endabschnitt des Kanülenrohrs ist in einem Kanülenrohrausschnitt angeordnet und dort kippbar gelagert.

Lateral der beispielhaften Innenkanüle ist im distalen Endabschnitt des Kanülenrohrs der erste Schlauch für die Befüllung des Cuffs angeordnet. Die beispielhafte Innenkanüle ist mittels des Anschlussmittels an dem Kanülenrohr befestigt. Die Innenkanüle umfasst eine Fensterung, die im Bereich der Siebung der Trachealkanüle angeordnet ist.

Der distaler Endabschnitt des beispielhaften Kanülenrohrs ist in dem Kanülenrohrausschnitt angeordnet und dort kippbar gelagert. Der beispielhaft kastenförmige, distale Endabschnitt umfasst eine superiore Fläche und eine dieser gegenüberliegend angeordnete, inferiore Fläche. Das beispielhafte Kanülenschild weist einen Kanülenrohrausschnitt auf, der von einer distalen Seite zu einer proximalen Seite durchgängig ist und in den der distale Endabschnitt anordenbar ist. Das Kanülenschild umfasst beispielhaft einen gradlinigen, den Kanülenrohrausschnitt begrenzenden ersten Abschnitt, welcher der superioren Fläche des distalen Endabschnittes zugeordnet ist. Weiterhin umfasst das Kanülenschild beispielhaft einen gradlinigen, den Kanülenrohrausschnitt begrenzenden, zweiten Abschnitt, welcher der inferioren Fläche des distalen Endabschnittes zugeordnet ist. Das Kanülenschild ist beispielhaft mittels eines Drehlagers am distalen Endabschnitt um eine medial-laterale Achse kippbar angeordnet. Das beispielhafte Kanülenschild umfasst laterale, den Kanülenrohrausschnitt begrenzenden Abschnitte, wobei das Drehlager zwischen den lateralen Abschnitten des Kanülenrohrausschnitts und lateralen Flächen des Endabschnitts angeordnet ist. Kippten distaler Endabschnitt und Kanülenschild zueinander wird ein Spalt zwischen diesen verringert.

Das beispielhafte Kanülenrohr ist in einen distalen Endabschnitt und einen trachealen Endabschnitt unterteilt, die an einer Kontaktstelle aneinander grenzen. Der distale Endabschnitt umfasst beispielhaft das Anschlussmittel für die Innenkanüle. Die beispielhafte Trachealkanüle umfasst eine vom distalen Ende zum trachealen Ende durchgehende Kanülenausnehmung mit einem gleichbleibenden Innendurchmesser. Die Kanülenausnehmung ist beispielhaft von einer Wandung, die das Kanülenrohr bildet, umschlossen, die einen ersten Außendurchmesser, der dem Kanülenschild zugeordnet ist. Die Wandung beziehungsweise das Kanülenrohr umfasst beispielhaft einen zweiten Außendurchmesser, der dem trachealen Ende zugeordnet ist. Der erste Außendurchmesser ist größer als der zweite Außendurchmesser und eine Mantelfläche der Wandung erstreckt sich konisch von dem ersten Außendurchmesser zum zweiten Außendurchmesser. Beispielhaft ist eine erste Wandstärke der Wandung an der Stelle des ersten Außendurchmessers größer als eine zweite Wandstärke der Wandung an der Stelle des zweiten Außendurchmessers. Da der Innendurchmesser gleichbleibend ausgestaltet ist verjüngt sich der der tracheale Endabschnitt nach tracheal konisch. Die Wandstärke des superioren und inferioren Bereichs des Kanülenrohrs, beziehungsweise des trachealen Endabschnitts, nimmt von distal nach tracheal kontinuierlich ab. Ebenso nimm die Wandstärke der lateralen Bereiche des Kanülenrohrs, beziehungsweise des trachealen Endabschnitts, von distal nach tracheal kontinuierlich ab. Der distale Endabschnitt des Kanülenrohrs erstreckt sich beispielhaft von inferior nach superior über einen ersten Außendurchmesser des trachealen Endabschnittes hinaus.

Beispielhaft ist der distale Endabschnitt ist zweiteilig ausgestaltet, wobei ein erster Teil beispielhaft materialeinheitlich mit dem trachealen Endabschnitt verbunden ist. Der zweite Teil kann formschlüssig und/oder materialschlüssig mit dem ersten Teil verbunden werden. Die Wandung weist beispielhaft eine erste Schlauchführung und eine zweite Schlauchführung auf, die auf der gegenüberliegenden Seite des Kanülenrohrs angeordnet ist. In der ersten Schlauchführung ist der erster Schlauch angeordnet und in der zweiten Schlauchführung ist der zweite Schlauch angeordnet. Der erste Schlauch und/oder der zweite Schlauch sind beispielhaft in einem Querschnitt linsenförmig ausgestaltet, wobei beide Hälften der Linsenform unterschiedliche Radien aufweisen. Die erste Schlauchführung mündet in eine erste Schlauchausnehmung des distalen Endabschnittes. Die zweite Schlauchführung mündet in eine zweite Schlauchausnehmung des distalen Endabschnittes. Eine beispielhafte Umhüllende, die beispielhaft unmittelbar an der Wandung anliegt, welche eine Mantelfläche der Wandung und den ersten Schlauch in der ersten Schlauchführung und den zweiten Schlauch in der zweiten Schlauchführung umhüllt, ist im Wesentlichen stetig. Zwischen der Mantelfläche und dem ersten und zweiten Schlauch ist beispielhaft im Wesentlichen keine Fuge und kein Versprung.

Beispielhaft sind die beiden Schlauchführungen in einem Bereich der Trachealkanüle angeordnet, die eine Materialanhäufung aufweist. Im Bereich des trachealen Endes der jeweiligen Schlauchführung weist die Trachealkanüle jeweils eine Materialanhäufung für auf, die wesentlich über die Wandstärke des umgebenen Bereiches herausragt. Die Schlauchführungen sind in der Materialanhäufung angeordnet. Eine Wandstärke im umgebenen Bereich der Materialanhäufungen der ersten und zweiten Schlauchführung ist dünner ausgestaltet insbesondere im Bereich des trachealen Endes der Schlauchführungen. Die Wandstärke nimmt bei der beispielhaften Trachealkanüle zum distalen Ende hin stetig zu. Die Wandstärke im Bereich der Materialanhäufung in Richtung der Längserstreckung der Trachealkanüle bleibt hingegen gleich. Hierdurch können die beispielhaften Schlauchführungen über die Längserstreckung der Trachealkanüle gleich tief im Material eingebettet sein. In einer Ausgestaltung ist vorgesehen, dass die Materialanhäufung zumindest abschnittsweise einen Radius, bevorzugt einen Radius der Mantelfläche, aufweist, der etwa gleich dem Radius des Kanülenrohrs etwa an der Grenze zum distalen Endabschnitt ist.

Beispielhaft gleicht die erste Schlauchführung in einen Querschnitt einem Oval-Segment. Weiterhin beispielhaft ist die zweite Schlauchführung in einem Querschnitt senkrecht zur Längserstreckung des Kanülenrohrs u-förmig ausgestaltet.

In einer beispielhaften Ausgestaltung des ersten Schlauches und/oder des zweiten Schlauches ist dieser in einem Querschnitt linsenförmig ausgestaltet. Beispielhaft ist der Querschnitt des Schlauches, besonders bevorzugt der Querschnitt der Mantelfläche des Schlauches, beidseitig konvex ausgebildet. Beispielsweise weist der Schlauch durch eine Teilung in Erstreckungsrichtung zwei Schlauchabschnitte auf, die im Querschnitt jeweils konvex ausgebildet sind und der Länge nach verbunden sind. Beispielhaft sind die konvexen Schlauchabschnitte unterschiedlich geformt. Der erste Schlauchabschnitt umfasst beispielhaft im Querschnitt einen ersten Radius R1 und der zweite Schlauchabschnitt umfasst im Querschnitt einen zweiten Radius R2, wobei beispielhaft der erste Radius R1 kleiner ist als der zweite Radius R2. Der Außenumfang des zweiten Schlauchabschnittes hat etwa den gleichen Radius R2 wie der Außenumfang des Kanülenrohrs, beispielsweise an der Grenze zum distalen Endabschnitt.

In einer Ausgestaltung ist vorgesehen, dass ein Obturator für eine Trachealkanüle umfassend einen Führungsstab, eine atraumatische tracheale Spitze, ein distales Griffstück und eine Hülse vorgesehen ist, wobei das Griffstück an einem distalen Ende des Führungsstabs angeordnet ist, wobei ein dem Führungsstab zugewandter Abschnitt des Griffstücks ein Gewinde umfasst, auf das die Hülse schraubbar ist.

Mittels des Obturators ist vorteilhaft ein atraumatisches Einführen der Trachealkanüle in die Trachea eines Benutzers möglich. Vorteilhaft ist der Obturator an eine Trachealkanüle angepasst. Bevorzugt ist zumindest die Spitze des Obturators, bevorzugt ein maximaler Durchmesser der Spitze, auf einen Innendurchmesser und/oder einen Außendurchmesser des Kanülenrohrs angepasst. Bevorzugt ist die Spitze klemmend zumindest in dem trachealen Endabschnitt der Trachealkanüle anordenbar. In einer Ausgestaltung ist vorgesehen, dass die Spitze ein gummielastisches Material umfasst. Beispielsweise umfasst die Spitze einen maximalen Durchmesser der etwa gleich oder größer einem Innendurchmesser des Kanülenrohrs ist, für das der Obturator vorgesehen ist. Vorteilhaft ist mittels der Hülse der Obturator zumindest teilweise aus dem Kanülenrohr herausziehbar. In einer Ausgestaltung ist vorgesehen, dass die Hülse mittels Drehung um ihre Längsachse translatorisch auf dem Griffstück bewegbar ist. In eine Trachealkanüle eingeführt kann die Hülse eine Kraft gegen die Trachealkanüle ausüben, um Obturator und Trachealkanüle relativ zueinander zu bewegen. Bevorzugt wirkt die Kraft auf das Kanülenrohr, weiter bevorzugt auf den distalen Endabschnitt des Kanülenrohrs. Weiter bevorzugt wirkt die Kraft ausschließlich auf das Kanülenrohr und bevorzugt nicht auf das Kanülenschild. Vorteilhaft wird hierdurch erreicht, dass Kanülenschild und Kanülenrohr nicht zueinander verkippen, wenn der Obturator aus dem Kanülenrohr gezogen wird.

Bevorzugt sind Führungsstab und Griffstück einstückig beziehungsweise monolithisch ausgestaltet. Weiter bevorzugt ist die Spitze auf den Führungsstab aufgerastet beziehungsweise formschlüssig gehalten. Weiter bevorzugt ist die Hülse mit Spiel auf dem Griffstück angeordnet. In einer Ausgestaltung ist vorgesehen, dass die Spitze einen maximalen Spitzendurchmesser aufweist, der größer ist als ein maximaler Stabdurchmesser des Führungsstabes.

In einer Ausgestaltung ist vorgesehen, dass am Obturator, bevorzugt am Griffstück, ein Bajonettverschlussteil angeordnet ist. Bevorzugt ist das Bajonettverschlussteil mit einem Bajonettverschlussteil der Trachealkanüle verbindbar.

In einer Ausgestaltung ist vorgesehen, dass das Gewinde am Griffstück abschnittsweise unterbrochen ist. Hierdurch wird eine möglichst geringe Reibung zwischen Hülse und Gewinde erzeugt. Bevorzugt sind je Gewindegang etwa zwei bis etwa vier, bevorzugt genau zwei Gewindeabschnitte auf dem Griffstück angeordnet. Bevorzugt ergänzen sich die Gewindeabschnitte beziehungsweise sind derart auf dem Griffstück angeordnet, dass ein Innengewinde der Hülse auf diese schraubbar ist. Bevorzugt ist das Griffstück zwischen den Gewindeabschnitten im Wesentlichen glatt ausgestaltet. In einer Ausgestaltung ist vorgesehen, dass das Gewinde ein Flachgewinde, ein Trapezgewinde oder ein Rundgewinde ist.

In einer Ausgestaltung ist vorgesehen, dass der Obturator bevorzugt dem Griffstück zugeordnet ein Bajonettverschlussteil aufweist. Bevorzugt ist das Bajonettverschlussteil mit dem Bajonettverschlussteil der Trachealkanüle, bevorzugt dem Kanülenschild, koppelbar. Vorteilhaft kann der Obturator derart sicher und bevorzugt fixiert in der Trachealkanüle gehalten werden. Bevorzugt ist der Bajonettverschluss von Obturator und Trachealkanüle lösbar, um den Obturator aus der Trachealkanüle zu entfernen.

In einer Ausgestaltung umfasst der Obturator eine distale Platte. Die distale Platte ist bevorzugt an einem distalen Ende des Führungsstabs angeordnet. Bevorzugt ist die distale Platte mit dem Führungsstab monolithisch ausgebildet. Die distale Platte verhindert vorteilhaft ein zu weites Einführen des Obturators in eine Trachealkanüle. Bevorzugt ist das Griffstück an der distalen Platte befestigbar.

In einer beispielhaften Ausgestaltung des Obturators zur atraumatischen Einführung der Trachealkanüle umfasst dieser einen Führungsstab, eine atraumatische trachealen Spitze aus einem gummielastischen Material und ein distales Griffstück. Das Griffstück ist beispielhaft an einem distalen Ende des Führungsstabs angeordnet. Ein dem Führungsstab zugewandter Abschnitt des Griffstücks umfasst beispielhaft ein Gewinde, auf das eine Hülse schraubbar ist. Das Gewinde ist beispielhaft als Flachgewinde ausgestaltet und nur teilweise ausgeführt beziehungsweise abschnittsweise unterbrochen. Die Spitze ist beispielhaft formschlüssig mit dem Führungsstab verbunden. Die Spitze weist beispielhaft einen maximale Spitzendurchmesser auf, der größer ist als ein maximaler Stabdurchmesser.

In einer weiteren beispielhaften Ausgestaltung umfasst der Obturator einen Führungsstab, eine atraumatische tracheale Spitze aus einem gummielastischen Material und eine distales Platte. Die distale Platte ist an einem distalen Ende des Führungsstabs angeordnet und mit diesem monolithisch ausgebildet.

In einer Ausgestaltung ist ein Set umfassend zumindest eine Trachealkanüle, bevorzugt eine oben beschriebene Trachealkanüle, und einen oben genannten Obturator vorgesehen, wobei eine Länge des Führungsstabes des Obturators an eine Länge des Kanülenrohrs der Trachealkanüle angepasst ist. Ein Set aus Trachealkanüle und Obturator ist vorteilhaft aufeinander eingestellt, wobei beispielsweise ein maximaler Spitzendurchmesser und/oder ein Außendurchmesser des Führungsstabes kleiner oder gleich einem maximalen Innendurchmesser des Kanülenrohrs ist. Weiterhin ist ein Krümmungsradius des Führungstabes etwa gleich einem Krümmungsradius des Kanülenrohrs.

In einer Ausgestaltung ist vorgesehen, dass die Spitze des Obturators einen Außendurchmesser beziehungsweise maximalen Spitzendurchmesser aufweist, der größer ist als ein Innendurchmesser des Kanülenrohrs an dem trachealen Ende. In einer Ausgestaltung ist vorgesehen, dass die Spitze des Obturators einen maximalen Spitzendurchmesser aufweist, der etwa gleich oder größer zumindest einem Innendurchmesser eines Kanülenrohrs, bevorzugt im Wesentlichen gleich oder größer als ein Außendurchmesser der Trachealkanüle an deren trachealen Ende ist. Besonders bevorzugt sind die Spitze des Obturators beziehungsweise dessen Oberfläche im eingesetzten Zustand mit dem Kanülenrohr beziehungsweise dessen Mantelfläche bündig.

Bevorzugt ist der Obturator in die Trachealkanüle derart einführbar, dass die Spitze am trachealen Ende der Trachealkanüle zumindest teilweise heraus ragt. Weiter bevorzugt ist die Spitze derart an das tracheale Ende der Trachealkanüle angepasst, dass diese einen maximalen Durchmesser aufweist, der etwa 0,1 mm bis etwa 1 mm größer ist als der Innendurchmesser der Trachealkanüle. Weiter bevorzugt ist der maximale Durchmesser der Spitze etwa gleich einem Außendurchmesser der Trachealkanüle an dem trachealen Ende. Vorteilhaft kann derart die Trachealkanüle mit eingesetzten Obturator atraumatisch in das Tracheostoma eingesetzt werden.

Insbesondere klemmt oder rastet die Spitze des Obturators im trachealen Endabschnitt der Trachealkanüle. Bevorzugt entspannt sich das gummielastische Material der Spitze beim Austritt aus dem distalen Ende, so dass ein Formschluss von Obturator und Trachealkanüle gegeben ist. Bevorzugt grenzt eine Oberfläche der Spitze an eine Mantelfläche der Trachealkanüle. Bevorzugt überlappt die Spitze nicht mit der Mantelfläche. In einer Ausgestaltung ist vorgesehen, dass bei in die Trachealkanüle eingeführten Obturator mittels Schrauben der Hülse auf dem Griffstück die Hülse eine Kraft gegen ein distales Ende der Trachealkanüle ausübbar ist. Mittels der Kraft kann die Spitze in die Trachealkanüle gezogen werden, wobei sich die Spitze bevorzugt verformt. Hierdurch wird das Entfernen des Obturators sicherer und komfortabler als wenn dieser manuell aus der Trachealkanüle herausgezogen wird.

In einer Ausgestaltung umfasst das Set eine Innenkanüle. Bevorzugt ist die Innenkanüle der Trachealkanüle entnommen, wenn die Trachealkanüle mittels des Obturators platziert wird.

In einer beispielhaften Ausgestaltung des Sets umfasst diese den Obturator und die Trachealkanüle. Auf das Gewinde des Griffstücks ist beispielhaft die Hülse aufgeschraubt, die im zusammengesetzten Zustand an den distalen Endabschnitt der Trachealkanüle angrenzt. Der Führungsstab ragt beispielhaft durch das Kanülenrohr, wobei die Spitze am trachealen Ende der Trachealkanüle hinaus ragt. Der maximale Spitzendurchmesser ist beispielhaft etwa gleich einem Innendurchmesser des Kanülenrohrs am trachealen Ende.

In einer Ausgestaltung ist ein Verfahren zum Herausziehen eines oben beschrieben Obturators aus einer Trachealkanüle vorgesehen, umfassend die Schritte:
- Schrauben einer Hülse auf einem Griffstück des Obturators gegen die Trachealkanüle,
- Schrauben der Hülse gegen den Widerstand der Trachealkanüle, wodurch zumindest der Führungsstab nach distal bewegt wird,
- Herausziehen des Obturators aus der Trachealkanüle.

In einer Ausgestaltung ist vorgesehen, dass der Obturator aus der Trachealkanüle herausgezogen wird, sobald die Spitze zumindest mittels Schrauben der Hülse teilweise in die Trachealkanüle eingezogen ist.

Weiterhin wird die Aufgabe mittels einer nicht beanspruchten Verwendung einer oben genannten Trachealkanüle zur atraumatischen Platzierung der Trachealkanüle in einer Trachea gelöst.

In einer Ausgestaltung ist vorgesehen, dass zumindest eine Schlauchführung zur Aufnahme eines Schlauches verwendet wird.
einer Ausgestaltung ist eine Verwendung zumindest einer oben genannten Trachealkanüle zur Aufnahme eines ersten Schlauches derart vorgesehen, dass ein Außendurchmesser des ersten Schlauches in radialer Erstreckung eines Kanülenrohrs kleiner oder etwa gleich einer Tiefe einer ersten Schlauchführung ist oder der erste Schlauch zumindest abschnittsweise eine Mantelfläche eines Kanülenrohrs derart ergänzt, dass ein Außenumfang der Mantelfläche des Kanülenrohrs weiter geführt ist, wobei der Außendurchmesser des ersten Schlauchs einem im Wesentlichen gleichen, größeren oder kleineren Radius als die Mantelfläche im Bereich der ersten Schlauchführung aufweist. In einer Ausführungsform ist vorgesehen, dass der ersten Schlauch in der Schlauchführung zur Bildung einer in einem Querschnitt im Wesentlichen stetigen Umhüllenden einer Mantelfläche einer Wandung eines Kanülenrohrs der Trachealkanüle und dem ersten Schlauch verwendet wird.

In einer Ausgestaltung ist eine Verwendung einer oben genannten Trachealkanüle mit einem Kanülenschild mit einem Kanülenrohrausschnitt vorgesehen, der sich von proximal nach distal erstreckt, wobei das Kanülenschild jeweils superior und inferior einen gradlinigen, den Kanülenrohrausschnitt begrenzenden Abschnitt aufweist und wobei das Kanülenschild ein Drehlager umfasst, zur kippbaren Anordnung an einem distalen Endabschnitt eines Kanülenrohrs, der eine superiore Fläche und einen dieser gegenüberliegenden, inferiore Fläche aufweist. Vorteil der Verwendung des Kanülenschildes ist, dass die Bewegungsfreiheit eines Patienten, insbesondere beim Nicken des Kopfes, erhöht wird. Dies trägt wesentlich zum Komfort der Trachealkanüle im täglichen Gebrauch bei.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
- Fig. 1: eine Trachealkanüle;
- Fig. 2: eine Trachealkanüle mit einer Innenkanüle;
- Fig. 3: einen Längsschnitt der Trachealkanüle aus Fig. 2;
- Fig. 4: eine Explosionsansicht einer Trachealkanüle;
- Fig. 5: eine Seitenansicht der Trachealkanüle 20;
- Fig. 6: eine Schnittansicht VI-VI aus Fig. 5;
- Fig. 7: eine Schnittansicht VII-VII aus Fig. 5;
- Fig. 8: eine Schnittansicht VIII-VIII aus Fig. 5;
- Fig. 9: ein Kanülenschild mit einem Teil eines Endabschnitts der Trachealkanüle;
- Fig. 10: ein Obturator;
- Fig. 11: ein Obturator in einer Trachealkanüle in einem Längsschnitt;
- Fig. 12: eine weitere Ausgestaltung eines Obturators; und
- Fig. 13: eine Schnittansicht des ersten Schlauches.

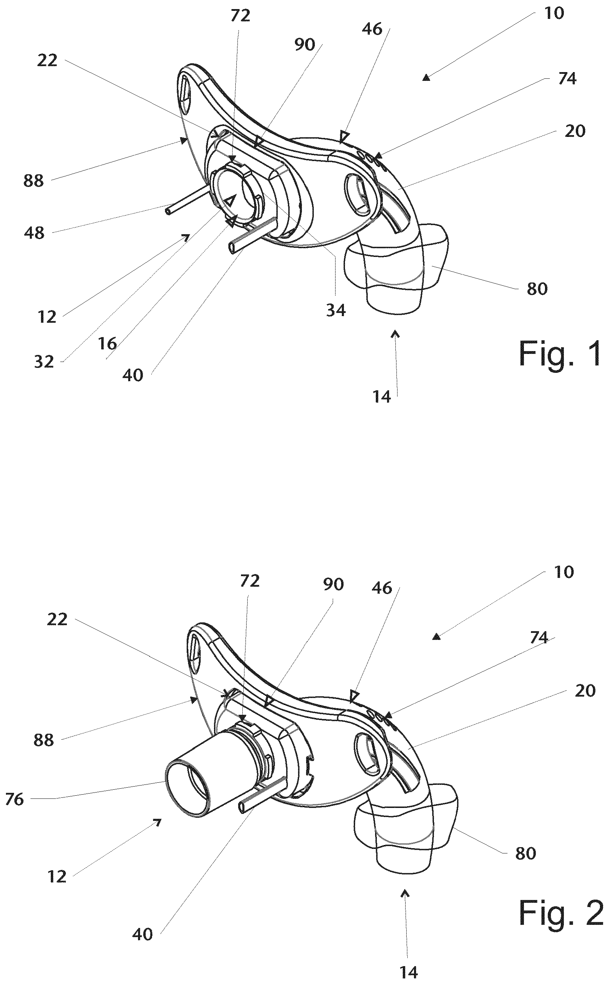

Fig. 1 zeigt eine Trachealkanüle 10 mit einem Kanülenrohr 20 und einem Kanülenschild 88. Die Trachealkanüle 10 umfasst einen ersten Schlauch 40 für eine subglottische Absaugung und einen zweiten Schlauch 48 zur Befüllung eines Cuffs 80. Der erste Schlauch 40 weist einen linsenförmigen Querschnitt auf. Der zweite Schlauch 48 weist einen runden Querschnitt auf. Das Kanülenrohr 20 umfasst auf einer distalen Seite 12 eine Öffnung 32 um dessen Rand 34 ein Anschlussmittel 72 angeordnet ist, mittels dem eine in Fig. 2 gezeigte Innenkanüle anschließbar ist. Weiterhin umfasst das Kanülenrohr 20 eine Siebung 74 im trachealen Endabschnitt 46. Ein distaler Endabschnitt 22 des Kanülenrohrs 20 ist in einem Kanülenrohrausschnitt 90 angeordnet und dort kippbar gelagert.

Fig. 2 zeigt die Trachealkanüle 10 aus Fig. 1 mit dem Kanülenrohr 20 und dem Kanülenschild 88 sowie einer Innenkanüle 76. Lateral der Innenkanüle 76 ist im distalen Endabschnitt 22 des Kanülenrohrs 20 der erste im Querschnitt linsenförmige Schlauch 40 für die Befüllung eines Cuffs 80 angeordnet. Die Innenkanüle 76 ist mittels des Anschlussmittels 72 an dem Kanülenrohr 20 befestigt. Weiterhin umfasst das Kanülenrohr 20 eine Siebung 74 im trachealen Endabschnitt 46. Der distaler Endabschnitt 22 des Kanülenrohrs 20 ist in dem Kanülenrohrausschnitt 90 angeordnet und dort kippbar gelagert.

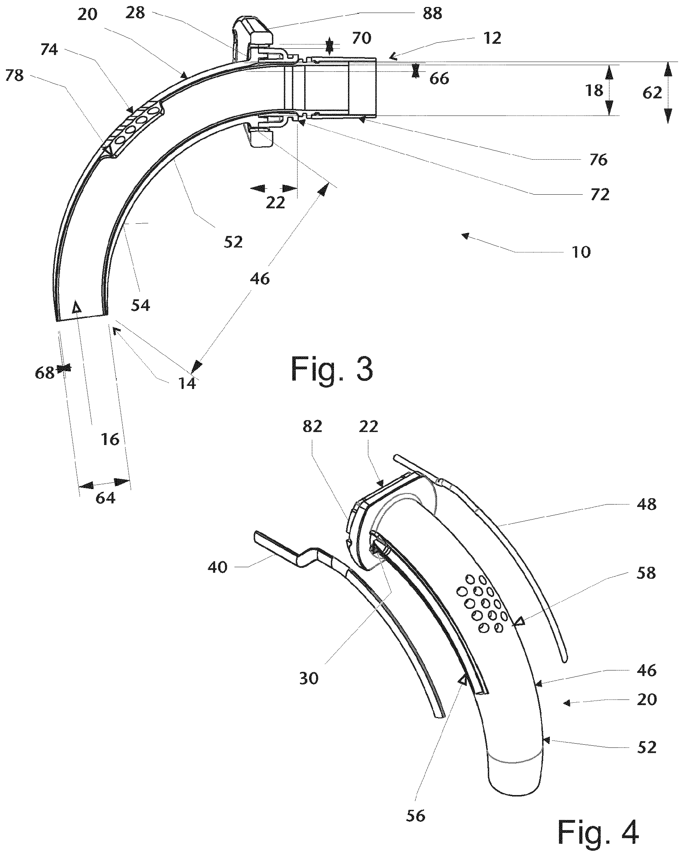

Fig. 3 zeigt einen Querschnitt der Trachealkanüle 10 aus Fig. 2. Das Kanülenrohr 20 ist in einen distalen Endabschnitt 22 und einen trachealen Endabschnitt 46 unterteilt, die an einer Kontaktstelle 28 aneinander grenzen. Der distale Endabschnitt 22 ist in dem Kanülenschild 88 gelagert. Kippten distaler Endabschnitt 22 und Kanülenschild 88 zueinander wird ein Spalt 70 zwischen diesen verringert. Der distale Endabschnitt 22 umfasst das Anschlussmittel 72 für die Innenkanüle 76. Die Innenkanüle 76 weist ein Fenster 78 auf, das der Siebung 74 der Trachealkanüle 10 zuordenbar ist. Die Trachealkanüle 10 umfasst eine vom distalen Ende 12 zum trachealen Ende 14 durchgehende Kanülenausnehmung 16 mit einem gleichbleibenden Innendurchmesser 18. Die Kanülenausnehmung 16 ist von einer Wandung 52, die das Kanülenrohr 20 bildet, umschlossen, die einen ersten Außendurchmesser 62, der dem Kanülenschild 88 zugeordnet ist. Die Wandung 52 beziehungsweise das Kanülenrohr 20 umfasst einen zweiten Außendurchmesser 64, der dem trachealen Ende 14 zugeordnet ist. Der erste Außendurchmesser 62 ist größer als der zweite Außendurchmesser 64 und eine Mantelfläche 54 der Wandung 52 erstreckt sich konisch von dem ersten Außendurchmesser 62 zum zweiten Außendurchmesser 64. Eine erste Wandstärke 66 der Wandung 52 an der Stelle des ersten Außendurchmessers 62 ist größer als eine zweite Wandstärke 68 der Wandung 52 an der Stelle des zweiten Außendurchmessers 64. Da der Innendurchmesser 18 gleichbleibend ausgestaltet ist, verjüngt sich der der tracheale Endabschnitt 46 nach tracheal konisch. Die Wandstärke 66, 68, des hier sichtbaren superioren und inferioren Bereichs des Kanülenrohrs 20 beziehungsweise des trachealen Endabschnitts 46, nimmt von distal nach tracheal kontinuierlich ab. Der distale Endabschnitt 22 des Kanülenrohrs 20 erstreckt sich von inferior nach superior über einen ersten Außendurchmesser 62 des trachealen Endabschnittes 46 hinaus.

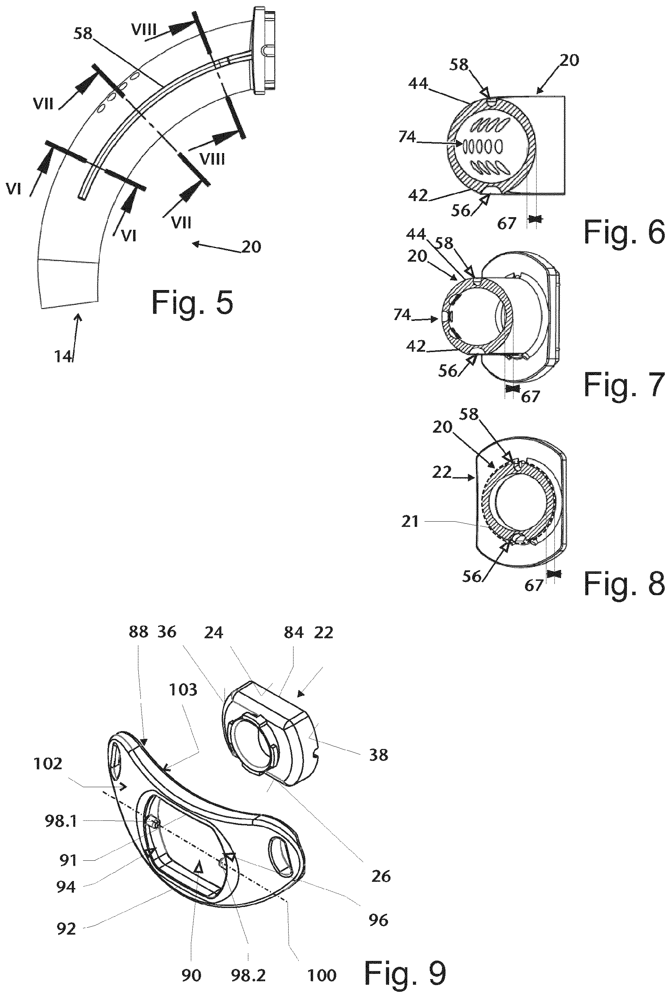

Fig. 4 zeigt ein Kanülenrohr 20 mit dem ersten Schlauch 40 und dem zweiten Schlauch 48 in einer Explosionsansicht. Der distale Endabschnitt 22 ist zweiteilig ausgestaltet, wobei ein erster Teil 82 materialeinheitlich mit dem trachealen Endabschnitt 46 verbunden ist. Der zweite Teil 84, der in Fig. 7 gezeigt ist, kann formschlüssig und/oder materialschlüssig mit dem ersten Teil 82 verbunden werden. Die Wandung 52 weist eine erste Schlauchführung 56 und eine zweite Schlauchführung 58 auf, die auf der gegenüberliegenden Seite des Kanülenrohrs 20 angeordnet ist. In der ersten Schlauchführung 56 ist der erster Schlauch 40 angeordnet und in der zweiten Schlauchführung 58 ist der zweite Schlauch 48 angeordnet. Die erste Schlauchführung 58 mündet in eine erste Schlauchausnehmung 30 des distalen Endabschnittes 22.

Fig. 5 zeigt eine Seitenansicht des Kanülenrohrs 20 ohne Schläuche 40 und 48.In Fig. 5 sind die Schnitte VI-VI, VII-VII und VIII-VIII in unterschiedlichen Abschnitten der Schlauchführung 58 angedeutet, die in Fig. 6, Fig. 7 und Fig. 8 gezeigt sind.

Fig. 6 zeigt einen Schnitt VI-VI der Trachealkanüle 20 aus Fig. 5 im Bereich des trachealen Endes 14 der zweiten Schlauchführung 58 mit Blick in Richtung der Siebung 74. Diese weist im Bereich der ersten Schlauchführung 56 und der zweiten Schlauchführung 58 jeweils Materialanhäufungen 42, 44, in denen die Schlauchführungen 56 und 58 angeordnet sind. Eine Wandstärke 67 im umgebenen Bereich der Materialanhäufungen 42, 44 der ersten und zweiten Schlauchführung 56 und 58 ist dünner ausgestaltet. Die Kanülenausnehmung 16 ist kreisrund ausgestaltet.

Fig. 7 zeigt einen Schnitt VII-VII der Trachealkanüle 20 aus Fig. 5 im Bereich der Siebung 74. Auch hier ist im Bereich der Schlauchführungen 56 und 58 eine Materialanhäufung 42, 44 angeordnet, die allerdings in Relation zur Wandstärke 67 weniger stark ausfällt, da die Wandstärke 67 im Bereich der Siebung größer ist als im Bereich des trachealen Endes.

Fig. 8 zeigt einen Schnitt VIII-VIII der Trachealkanüle 20 aus Fig. 5 an der Grenze zum distalen Endabschnitt 22. Die Wandstärke 67 über den Außenumfang 21 des Trachealkanüle 20 etwa gleich dick. Da die Wandstärke 67 zum distalen Endabschnitt 22 hin größer wird, ist diese dort ausreichend dick um die Schlauchführungen 56 und 58 beziehungsweise die hier nicht gezeigten Schläuche aufzunehmen.

In Fig. 6, Fig. 7 und Fig. 8 ist insbesondere erkennbar, dass die erste Schlauchführung 56 einen Querschnitt aufweist, der einem Oval-Segment gleicht. Weiterhin ist die zweite Schlauchführung 58 u-förmig ausgestaltet. Weiterhin ist den Fig 6, Fig. 7 und Fig. 8 zu entnehmen, dass die Wandstärke 67, des lateralen Bereichs des Kanülenrohrs 20 beziehungsweise des trachealen Endabschnitts 46, der in Fig. 2 und Fig. 3 bezeichnet ist, von distal nach tracheal kontinuierlich abnimmt.

Fig. 9 zeigt das Kanülenschild 88 und einen zweiten Teil 84 des distalen Endabschnittes 22 in eine Explosionsansicht. Der kastenförmige, distale Endabschnitt 22 umfasst eine superiore Fläche 24 und eine dieser gegenüberliegend angeordnete, inferiore Fläche 26. Das Kanülenschild 88 weist einen Kanülenrohrausschnitt 90 auf, der von einer distalen Seite 102 zu einer proximalen Seite 103 durchgängig ist und in den der distale Endabschnitt 22 anordenbar ist. Das Kanülenschild 88 umfasst einen gradlinigen, den Kanülenrohrausschnitt 90 begrenzenden ersten Abschnitt 91, welcher der superioren Fläche 24 des distalen Endabschnittes 22 zugeordnet ist. Weiterhin umfasst das Kanülenschild 88 einen gradlinigen, den Kanülenrohrausschnitt 90 begrenzenden, zweiten Abschnitt 92, welcher der inferioren Fläche 26 des distalen Endabschnittes 22 zugeordnet ist. Das Kanülenschild 88 ist mittels eines Drehlagers 98.1 und 98.2 am distalen Endabschnitt 22 um eine medial-laterale Achse 100 kippbar angeordnet. Das Kanülenschild 88 umfasst laterale, den Kanülenrohrausschnitt 90 begrenzende Abschnitte 94, 96, wobei das Drehlager 98 zwischen den lateralen Abschnitten 94, 96 des Kanülenrohrausschnitts 90 und lateralen Flächen 36, 38 des Endabschnitts 22 angeordnet ist.

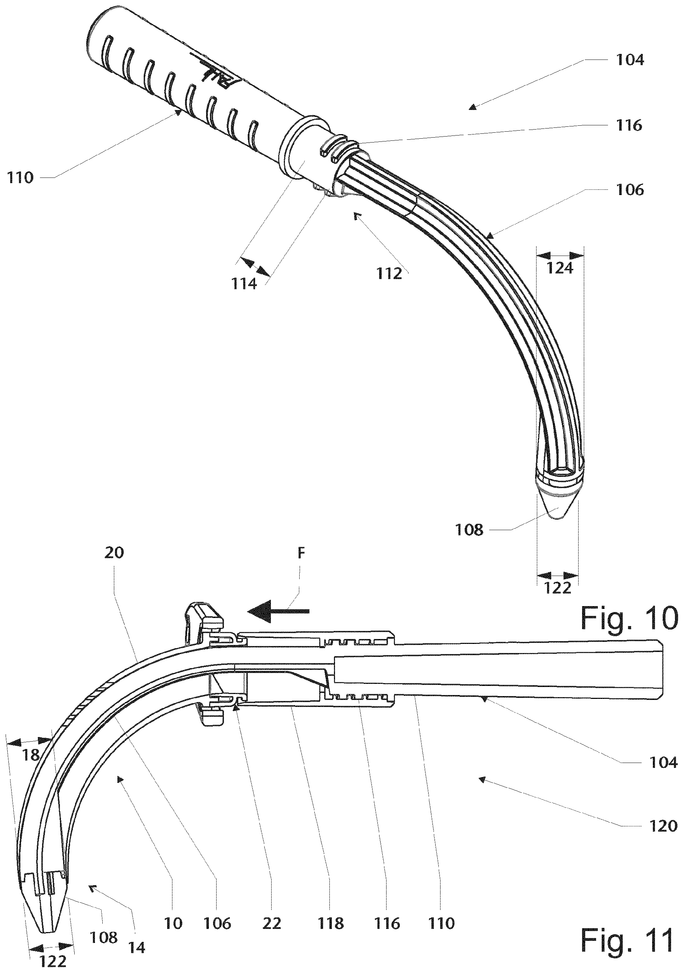

Fig. 10 zeigt einen Obturator 104 zur atraumatischen Einführung der Trachealkanüle 10. Dieser umfasst einen Führungsstab 106, eine atraumatische tracheale Spitze 108 aus einem gummielastischen Material, ein distales Griffstück 110. Das Griffstück 110 ist an einem distalen Ende 112 des Führungsstabs 106 angeordnet und mit diesem monolithisch ausgebildet. Ein dem Führungsstab 106 zugewandter Abschnitt 114 des Griffstücks 110 umfasst ein Gewinde 116, auf das eine Hülse 118 schraubbar ist, die in Fig. 11 gezeigt ist. Das Gewinde 116 ist als Flachgewinde ausgestaltet und nur teilweise ausgeführt beziehungsweise abschnittsweise unterbrochen. Die Spitze 108 ist formschlüssig mit dem Führungsstab 106 verbunden. Die Spitze 108 weist einen maximale Spitzendurchmesser 122 auf, der größer ist als ein maximaler Stabdurchmesser 124.

Fig. 11 zeigt ein Set 120 umfassend den Obturator 104 aus Fig. 10 und die Trachealkanüle 10 aus Fig. 1 im zusammengesetzten Zustand in einem Längsschnitt. Auf das Gewinde 116 des Griffstücks 110 ist die Hülse 118 aufgeschraubt, die an den distalen Endabschnitt 22 der Trachealkanüle 10 angrenzt. Der Führungsstab 106 ragt durch das Kanülenrohr 20, wobei die Spitze 108 am trachealen Ende 14 der Trachealkanüle 10 hinaus ragt. Der maximale Spitzendurchmesser 122 ist etwa gleich einem Innendurchmesser 18 des Kanülenrohrs 20 am trachealen Ende 14.

Die Hülse 118 kann gegen den distalen Endabschnitt 22 geschraubt werden und somit eine Kraft F aus diesen ausüben. Durch die Kraft F werden Trachealkanüle 10 und Obturator 104 relativ zueinander bewegt, wobei die Spitze 108 in das Kanülenrohr 20 zurück gezogen wird.

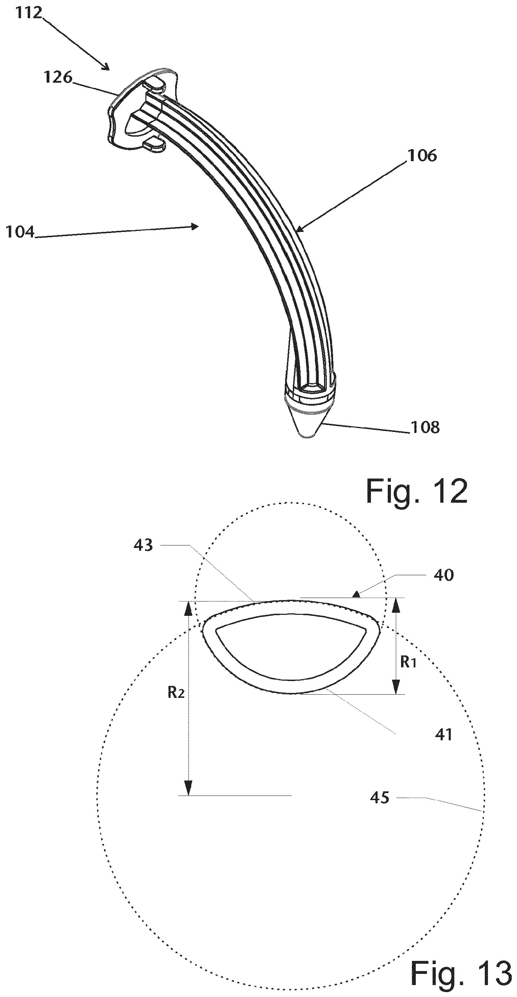

Fig. 12 zeigt eine weitere Ausgestaltung eines Obturators 104. Dieser umfasst einen Führungsstab 106, eine atraumatische tracheale Spitze 108 aus einem gummielastischen Material und eine distales Platte 126. Die distale Platte 126 ist an einem distalen Ende 112 des Führungsstabs 106 angeordnet und mit diesem monolithisch ausgebildet. Die distale Platte 126 verhindert ein zu weites Einführen des Obturators 104 in eine Trachealkanüle 20, die beispielswiese in Fig. 1 zu sehen ist. Die Spitze 108 ist formschlüssig mit dem Führungsstab 106 verbunden. Die Spitze 108 weist einen maximale Spitzendurchmesser 122 auf, der größer ist als ein maximaler Stabdurchmesser 124.

Fig. 13 zeigt den ersten Schlauch 40 in einem Querschnitt. Der erste Schlauch 40 ist im Querschnitt linsenförmig ausgestaltet, wobei konvexe Schlauchabschnitte 41, 43 unterschiedlich geformt sind. Der erste Schlauchabschnitt 41 umfasst im Querschnitt einen ersten Radius R1 und der zweite Schlauchabschnitt 43 umfasst im Querschnitt einen zweiten Radius R2, wobei der erste Radius R1 kleiner ist als der zweite Radius R2. Der Außenumfang 45 des zweiten Schlauchabschnittes 45 hat etwa den gleichen Radius R2 wie der Außenumfang 21 des Kanülenrohrs 20, der aus Fig. 8 entnehmbar ist, an der Grenze zum distalen Endabschnitt 22.

Vorteilhaft kann mittels der vorgeschlagenen Trachealkanüle und dem vorgeschlagenen Obturator ein Einführen der Trachealkanüle wesentlich gewebeschonender vorgenommen werden. Auch der Tragekomfort wird durch die vorgeschlagene Trachealkanüle wesentlich verbessert.

## Patentansprüche

1. Trachealkanüle (10) zumindest umfassend ein Kanülenrohr (20) und ein Kanülenschild (88), wobei das Kanülenrohr (20) einen distalen Endabschnitt (22), dem das Kanülenschild (88) zugeordnet ist, und einen trachealen Endabschnitt (46) umfasst, der in einer Trachea anordenbar ist,
wobei dem Kanülenrohr (20) ein erster Außendurchmesser (62) am Kanülenschild (88) zugeordnet ist,
wobei dem Kanülenrohr (20) ein zweiter Außendurchmesser (64) an einem trachealen Ende (14) zugeordnet ist,
wobei das Kanülenrohr (20) eine von einem distalen Ende (12) zu dem trachealen Ende (14) durchgehende Kanülenausnehmung (16) mit einem gleichbleibenden Innendurchmesser (18) umfasst, wobei eine Wandung (52) zumindest des trachealen Endabschnitts (46) eine erste Wandstärke (66) angrenzend an den distalen Endabschnitt (22) und eine zweite Wandstärke (68) am trachealen Ende (14) umfasst, wobei die erste Wandstärke (66) der Wandung (52) an der Stelle des ersten Außendurchmessers (62) größer als eine zweite Wandstärke (68) der Wandung (52) an der Stelle des zweiten Außendurchmessers (64) ist, wobei die erste Wandstärke (66) größer als die zweite Wandstärke (68) ist und die Wandstärke (66, 68) des trachealen Endabschnitts (46) zum trachealen Ende (14) hin kontinuierlich abnimmt.

2. Trachealkanüle (10) gemäß Anspruch 1, **dadurch gekennzeichnet, dass** eine Wandung (52) des Kanülenrohrs (20) zumindest des trachealen Endabschnitts (46) in einem Querschnitt senkrecht zu einer Erstreckung des Kanülenrohrs (20) zumindest einseitig eine Materialanhäufung (42, 44) aufweist.

3. Trachealkanüle (10) nach einem oder mehreren der vorhergehenden Ansprüche, da-
durch gekennzeichnet, dass ein Außenumfang (21) der Trachealkanüle (10) in einem Querschnitt senkrecht zu einer Erstreckung des Kanülenrohrs (20) zumindest abschnittsweise im Wesentlichen oval ist.

4. Trachealkanüle (10) nach einem oder mehreren der vorhergehenden Ansprüche, da-
durch gekennzeichnet, dass diese zumindest eine erste Schlauchführung (56) umfasst, die sich von einer distalen Seite (102) des Kanülenschilds (88) zumindest bis zu einem Cuff (80) erstreckt, welches dem trachealen Ende (14) zugeordnet ist.

5. Trachealkanüle (10) nach Anspruch 4, **dadurch gekennzeichnet, dass** zumindest die
erste Schlauchführung (56) zumindest abschnittsweise als Nut in der Wandung (52) des trachealen Endabschnitts (46) ausgebildet ist.

6. Trachealkanüle (10) nach einem oder mehreren der Ansprüche 4 bis 5, **dadurch gekennzeichnet, dass** zumindest die erste Schlauchführung (56) in einem verdickten Bereich der Wandung (52) des trachealen Endabschnitts (46) ausgebildet ist.

7. Trachealkanüle (10) nach einem oder mehreren der Ansprüche 4 bis 6, **dadurch gekennzeichnet, dass** ein erster Schlauch (40) zumindest teilweise in der ersten Schlauchführung (56) angeordnet ist.

8. Trachealkanüle (10) nach Anspruch 7, **dadurch gekennzeichnet, dass** der erste Schlauch (40) in einem Querschnitt senkrecht zu einer Erstreckung des Schlauches (40) oval, linsenförmig oder kreisrund ausgestaltet ist.

9. Trachealkanüle (10) nach einem oder mehreren der Ansprüche 7 bis 8, **dadurch gekennzeichnet, dass** der Außendurchmesser des ersten Schlauches (40) in radialer Erstreckung des Kanülenrohrs (20) kleiner oder etwa gleich einer Tiefe der ersten Schlauchführung (56) ist.

10. Trachealkanüle (10) nach einem oder mehreren der vorhergehenden Ansprüche, **dadurch gekennzeichnet, dass** diese zumindest eine zweite Schlauchführung (58) umfasst, die sich von einer distalen Seite (102) des Kanülenschilds (88) zumindest bis zum Cuff (80) erstreckt und in der ein zweiter Schlauch (48) geführt ist.

11. Trachealkanüle (10) nach Anspruch 10, **dadurch gekennzeichnet, dass** der zweite Schlauch (48) ein Füllschlauch für das Cuff (80) ist, der sich in das Cuff (80) hinein erstreckt.

## Claims

1. A tracheal cannula (10) at least comprising a cannula tube (20) and a cannula shield (88), wherein the cannula tube (20) comprises a distal end portion (22), to which the cannula shield (88) is assigned, and a tracheal end portion (46), which is able to be arranged in a trachea, wherein a first external diameter (62) is assigned to the cannula tube (20) at the cannula shield (88), wherein a second external diameter (64) is assigned to the cannula tube (20) at a tracheal end (14), wherein the cannula tube (20) comprises a cannula lumen (16) which is continuous from a distal end (12) to the tracheal end (14) and has a constant internal diameter (18), wherein a wall (52) at least of the tracheal end portion (46) comprises a first wall thickness (66) which is contiguous with the distal end portion (22), and a second wall thickness (68) at the tracheal end (14), wherein the first wall thickness (66) of the wall (52) at the site of the first external diameter (62) is greater than a second wall thickness (68) of the wall (52) at the site of the second external diameter (64),wherein the first wall thickness (66) is greater than the second wall thickness (68), and the wall thickness (66, 68) of the tracheal end portion (46) reduces continuously towards the tracheal end (14).

2. The tracheal cannula (10) according to Claim 1, **characterized in that** a wall (52) of the cannula tube (20) at least of the tracheal end portion (46) has at least on one side a material buildup (42, 44) in a cross-section perpendicularly to an extent of the cannula tube (20).

3. The tracheal cannula (10) according to one or more of the preceding claims, **characterized in that** an outer circumference (21) of the tracheal cannula (10) is at least partially substantially oval in a cross-section perpendicularly to an extent of the cannula tube (20).

4. The tracheal cannula (10) according to one or more of the preceding claims, **characterized in that** this comprises at least a first tube guide (56), which extends from a distal side (102) of the cannula shield (88) at least up to a cuff (80), which is assigned to the tracheal end (14).

5. The tracheal cannula (10) according to Claim 4, **characterized in that** at least the first tube guide (56) is formed at least partially as a groove in the wall (52) of the tracheal end portion (46).

6. The tracheal cannula (10) according to one or more of Claims 4 to 5, **characterized in that** at least the first tube guide (56) is formed in a thickened region of the wall (52) of the tracheal end portion (46).

7. The tracheal cannula (10) according to one or more of Claims 4 to 6, **characterized in that** a first tube (40) is arranged at least partially in the first tube guide (56).

8. The tracheal cannula (10) according to Claim 7, **characterized in that** the first tube (40) is configured in an oval, lenticular or circular manner in a cross-section perpendicularly to an extent of the tube (40).

9. The tracheal cannula (10) according to one or more of Claims 7 to 8, **characterized in that** the external diameter of the first tube (40) is smaller than or approximately equal to a depth of the first tube guide (56) in radial extent of the cannula tube (20).

10. The tracheal cannula (10) according to one or more of the preceding claims, **characterized in that** it comprises at least a second tube guide (58), which extends from a distal side (102) of the cannula shield (88) at least up to the cuff (80) and in which a second tube (48) is guided.

11. The tracheal cannula (10) according to Claim 10, **characterized in that** the second tube (48) is a filling tube for the cuff (80), which extends into the cuff (80).

## Revendications

1. Canule trachéale (10) comprenant au moins un tube de canule (20) et un embout de canule (88), le tube de canule (20) comportant une partie d'extrémité distale (22) à laquelle est associé l'embout de canule (88), et une partie d'extrémité trachéale (46) pouvant être placée dans une trachée,
le tube de canule (20) présentant un premier diamètre extérieur (62) au niveau de l'embout de canule (88),
l'embout de canule (20) présentant un deuxième diamètre extérieur (64) au niveau d'une extrémité trachéale (14),
le tube de canule (20) comprenant un évidement de canule (16) s'étendant de manière continue d'une extrémité distale (12) à l'extrémité trachéale (14) avec un diamètre intérieur constant (18), une paroi (52) d'au moins la partie d'extrémité trachéale (46) présente une première épaisseur de paroi (66) adjacente à la partie d'extrémité distale (22) et une deuxième épaisseur de paroi (68) au niveau de l'extrémité trachéale (14), la première épaisseur de paroi (66) de la paroi (52) à l'emplacement du premier diamètre extérieur (62) étant supérieure à une deuxième épaisseur de paroi (68) de la paroi (52) au niveau du deuxième diamètre extérieur (64), la première épaisseur de paroi (66) étant supérieure à la deuxième épaisseur de paroi (68) et l'épaisseur de paroi (66, 68) de la partie d'extrémité trachéale (46) diminuant de manière continue en direction de l'extrémité trachéale (14).

2. Canule trachéale (10) selon la revendication 1, **caractérisée en ce qu'**une paroi (52) du tube de canule (20), au moins au niveau de la partie d'extrémité trachéale (46), présente, dans une section transversale perpendiculaire à l'axe du tube de canule (20), au moins d'un côté (25), une accumulation de matière (42, 44).

3. Canule trachéale (10) selon une ou plusieurs des revendications précédentes, **caractérisée en ce qu'**une circonférence extérieure (21) de la canule trachéale (10), dans une section transversale perpendiculaire à la direction d'extension du tube de canule (20), est sensiblement ovale au moins par sections.

4. Canule trachéale (10) selon une ou plusieurs des revendications précédentes, **caractérisée en ce qu'**elle comprend au moins un premier guide-tube (56) qui s'étend depuis un côté distal (102) de l'embout de canule (88) au moins jusqu'à un ballonnet (80) associé à l'extrémité trachéale (14).

5. Canule trachéale (10) selon la revendication 4, **caractérisée en ce qu'**au moins le premier guide-tube (56) est réalisé, au moins par sections, sous la forme d'une rainure dans la paroi (52) de la partie d'extrémité trachéale (46).

6. Canule trachéale (10) selon l'une ou plusieurs des revendications 4 à 5, **caractérisée en ce qu'**au moins le premier guide-tube (56) est formé dans une zone épaissie de la paroi (52) de la partie d'extrémité trachéale (46).

7. Canule trachéale (10) selon l'une ou plusieurs des revendications 4 à 6, **caractérisée en ce qu'**un premier tube (40) est disposé au moins partiellement dans le premier guide-tube (56).

8. Canule trachéale (10) selon la revendication 7, **caractérisée en ce que** le premier tube (40) présente, dans une section transversale perpendiculaire à une direction d'extension du tube (40), une forme ovale, lenticulaire ou circulaire.

9. Canule trachéale (10) selon l'une ou plusieurs des revendications 7 à 8, **caractérisée en ce que** le diamètre extérieur du premier tube (40), dans le sens radial du tube de canule (20), est inférieur ou approximativement égal à la profondeur du premier guide-tube (56).

10. Canule trachéale (10) selon une ou plusieurs des revendications précédentes, **caractérisée en ce qu'**elle comprend au moins un deuxième guide-tube (58) qui s'étend depuis un côté distal (102) de l'embout de canule (88) jusqu'à au moins le ballonnet (80) et dans lequel est guidé un deuxième tube (48).

11. Canule trachéale (10) selon la revendication 10, **caractérisée en ce que** le deuxième tube (48) est un tube de remplissage pour le ballonnet (80), qui s'étend à l'intérieur du ballonnet (80).
